# EUROPEAN PATENT SPECIFICATION

(11) **EP 3 497 451 B1**
(45) Date of publication and mention of the grant of the patent: **18.06.2025**
(21) Application number: 17752362.8
(22) Date of filing: 08.08.2017
(51) Int. Cl.: G01N 33/68, G01N 33/74

(54) **HISTONES AND/OR PROADM AS MARKERS INDICATING AN ADVERSE EVENT**
HISTONE UND/ODER PROADM ALS MARKER ZUR ANZEIGE EINES UNERWÜNSCHTEN EREIGNISSES
HISTONES ET/OU PROADM COMME MARQUEURS INDIQUANT UN ÉVÉNEMENT DÉFAVORABLE

(30) Priority: 09.08.2016 EP 16183391
(43) Date of publication of application: 19.06.2019
(73) Proprietor: B.R.A.H.M.S GmbH, 16761 Hennigsdorf (DE)
(72) Inventor: ZIERA, Tim, 14129 Berlin (DE); SCHÖNICHEN, André, San Francisco, CA 94122 (US); INCAMPS, Anne, 30290 Saint Victor la Coste (FR); KROP, Manne, 13509 Berlin (DE); CURDT, Ingo, 13503 Berlin (DE); CHARLES, Pierre-Emmanuel, 21000 Dijon (FR)
(74) Representative: Vossius & Partner Patentanwälte Rechtsanwälte mbB
(86) International application number: PCT/EP2017/070112
(87) International publication number: WO 2018/029214

(56) References cited:
- EP-A2- 1 619 505
- EP-A2- 1 619 505
- EP-B1- 1 619 505
- WO-A1-2010/009965
- WO-A1-2010/009965
- WO-A1-2012/059477
- WO-A1-2012/059477
- MATTHEW E. KUTCHER ET AL: "Extracellular histone release in response to traumatic injury", JOURNAL OF TRAUMA AND ACUTE CARE SURGERY, vol. 73, no. 6, 1 December 2012 (2012-12-01), US, pages 1389 - 1394, XP055311367, ISSN: 2163-0755, DOI: 10.1097/TA.0b013e318270d595
- MICHAEL LIEMBO EKANEY ET AL: "Impact of plasma histones in human sepsis and their contribution to cellular injury and inflammation", CRITICAL CARE, BIOMED CENTRAL LTD., LONDON, GB, vol. 18, no. 5, 24 September 2014 (2014-09-24), pages 543, XP021201659, ISSN: 1364-8535, DOI: 10.1186/S13054-014-0543-8
- SIMON T. ABRAMS ET AL: "Circulating Histones Are Mediators of Trauma-associated Lung Injury", AMERICAN JOURNAL OF RESPIRATORY AND CRITICAL CARE MEDICINE., vol. 187, no. 2, 15 January 2013 (2013-01-15), US, pages 160 - 169, XP055288777, ISSN: 1073-449X, DOI: 10.1164/rccm.201206-1037OC
- MIHAEL POTOCKI ET AL: "Mid-Regional Pro-Adrenomedullin in Acute Heart Failure: A Better Biomarker or Just Another Biomarker?", CURRENT HEART FAILURE REPORTS, CURRENT SCIENCE INC, NEW YORK, vol. 9, no. 3, 26 June 2012 (2012-06-26), pages 244 - 251, XP035091322, ISSN: 1546-9549, DOI: 10.1007/S11897-012-0096-6
- FERRUH ARTUNC ET AL: "Plasma Concentrations of the Vasoactive Peptide Fragments Mid-Regional Pro-Adrenomedullin, C-Terminal Pro-Endothelin 1 and Copeptin in Hemodialysis Patients: Associated Factors and Prediction of Mortality", PLOS ONE, vol. 9, no. 1, 22 January 2014 (2014-01-22), pages e86148, XP055312685, DOI: 10.1371/journal.pone.0086148
- MEINDERS A ET AL: "Preoperative serum levels of mid-regional adrenomedullin and NTproBNP predict postoperative major adverse cardiovascular events and organ failures", EUROPEAN HEART JOURNAL, OXFORD UNIVERSITY PRESS, GB, vol. 33, no. Suppl. 1, 1 August 2012 (2012-08-01), pages 130, XP009192208, ISSN: 0195-668X
- KARIN C.A.A. WILDHAGEN ET AL: "Extracellular histone H3 levels are inversely correlated with antithrombin levels and platelet counts and are associated with mortality in sepsis patients", THROMBOSIS RESEARCH, vol. 136, no. 3, 1 September 2015 (2015-09-01), US, pages 542 - 547, XP055312885, ISSN: 0049-3848, DOI: 10.1016/j.thromres.2015.06.035
- DE LA TORRE-PRADOS MARIA V ET AL: "Mid-regional pro-adrenomedullin as prognostic biomarker in septic shock", MINERVA ANESTESIOLOGICA : GIORNALE ITALIANO DI ANESTESIA E DI ANALGESIA, SOCIETÀ ITALIANA DI ANESTESIOLOGIA, IT, vol. 82, no. 7, 1 July 2016 (2016-07-01), pages 760 - 766, XP009192235, ISSN: 1827-1596
- MATTHEW E. KUTCHER ET AL: "Extracellular histone release in response to traumatic injury", JOURNAL OF TRAUMA AND ACUTE CARE SURGERY, vol. 73, no. 6, 1 December 2012 (2012-12-01), US, pages 1389 - 1394, XP055311367, ISSN: 2163-0755, DOI: 10.1097/TA.0b013e318270d595
- MICHAEL LIEMBO EKANEY ET AL: "Impact of plasma histones in human sepsis and their contribution to cellular injury and inflammation", CRITICAL CARE, BIOMED CENTRAL LTD., LONDON, GB, vol. 18, no. 5, 24 September 2014 (2014-09-24), pages 543, XP021201659, ISSN: 1364-8535, DOI: 10.1186/S13054-014-0543-8
- SIMON T. ABRAMS ET AL: "Circulating Histones Are Mediators of Trauma-associated Lung Injury", AMERICAN JOURNAL OF RESPIRATORY AND CRITICAL CARE MEDICINE., vol. 187, no. 2, 15 January 2013 (2013-01-15), US, pages 160 - 169, XP055288777, ISSN: 1073-449X, DOI: 10.1164/rccm.201206-1037OC
- MIHAEL POTOCKI ET AL: "Mid-Regional Pro-Adrenomedullin in Acute Heart Failure: A Better Biomarker or Just Another Biomarker?", CURRENT HEART FAILURE REPORTS, CURRENT SCIENCE INC, NEW YORK, vol. 9, no. 3, 26 June 2012 (2012-06-26), pages 244 - 251, XP035091322, ISSN: 1546-9549, DOI: 10.1007/S11897-012-0096-6
- FERRUH ARTUNC ET AL: "Plasma Concentrations of the Vasoactive Peptide Fragments Mid-Regional Pro-Adrenomedullin, C-Terminal Pro-Endothelin 1 and Copeptin in Hemodialysis Patients: Associated Factors and Prediction of Mortality", PLOS ONE, vol. 9, no. 1, 22 January 2014 (2014-01-22), pages e86148, XP055312685, DOI: 10.1371/journal.pone.0086148
- MEINDERS A ET AL: "Preoperative serum levels of mid-regional adrenomedullin and NTproBNP predict postoperative major adverse cardiovascular events and organ failures", EUROPEAN HEART JOURNAL, OXFORD UNIVERSITY PRESS, GB, vol. 33, no. Suppl. 1, 1 August 2012 (2012-08-01), pages 130, XP009192208, ISSN: 0195-668X
- KARIN C.A.A. WILDHAGEN ET AL: "Extracellular histone H3 levels are inversely correlated with antithrombin levels and platelet counts and are associated with mortality in sepsis patients", THROMBOSIS RESEARCH, vol. 136, no. 3, 1 September 2015 (2015-09-01), US, pages 542 - 547, XP055312885, ISSN: 0049-3848, DOI: 10.1016/j.thromres.2015.06.035
- DE LA TORRE-PRADOS MARIA V ET AL: "Mid-regional pro-adrenomedullin as prognostic biomarker in septic shock", MINERVA ANESTESIOLOGICA : GIORNALE ITALIANO DI ANESTESIA E DI ANALGESIA, SOCIETÀ ITALIANA DI ANESTESIOLOGIA, IT, vol. 82, no. 7, 1 July 2016 (2016-07-01), pages 760 - 766, XP009192235, ISSN: 1827-1596

## Description

The present invention relates to the prognosis of mortality of a subject, wherein said subject is a critically ill patient. The invention relates to a method that comprises determining a level of at least one histone, particularly histone H2B, H4, H2A and/or H3, in a blood, blood plasma, or blood serum sample of the subject, and wherein the level of at least one histone is indicative of the mortality of said subject; and determining a level of proadrenomedullin (proADM) in the sample of the subject, and wherein the level of proADM is indicative of the mortality of said subject. The invention further relates to the use of a kit for carrying out the methods of the invention.

### Background of the invention

The intensive care unit (ICU) in a hospital is usually the unit with the most critically ill patients and the highest mortality rates (Kaneko-Wada Fde, Dominguez-Cherit et al. 2015).It is a very expensive component for any healthcare system, mainly due to long stays on the ICU, modern and costly technologies and the overall complexity of intensive care (Halpern and Pastores 2010). Despite high efforts of intensive care medicine, mortality rates on ICUs range from 6.4 % up to 40 %, depending on the analyzed patient population and their status of severity (Mayr, Dunser et al. 2006). Major causes of adverse outcome and death on the ICU are related to failing organs like the multiple organ dysfunction syndrome (MODS) and to sepsis (Vincent 2008; Ferreira and Sakr 2011). For the sub-group of septic patients, the mortality rate in Europe is even higher, ranging between 27 % and 54 % (Vincent 2008).

Kutcher et al. (2012) reports on extracellular histone release in response to traumatic injury. Ekaney et al. (2014) relates to Impact of plasma histones in human sepsis and their contribution to cellular injury and inflammation. Abrams et al. (2012) teaches that circulating histones are mediators of trauma-associated lung injury. Potocki et al. (2012) teaches that MR-proADM is a biomarker for acute heart failure. Artunc et al. (2014) relates to the role of MR-proADM as predictor of mortality in in hemodialysis patients. Meinders et al. (2012) reports that preoperative serum levels of MR-proADM and NTproBNP predict postoperative major adverse cardiovascular events and organ failures. Wildhagen et al. (2015) reports that extracellular histone H3 levels are associated with mortality in sepsis patients. De La Torre-Prados et al. (2016) teaches MR-proADM as prognostic biomarker in septic shock. WO 2012/059477 A1 relates to a method for the risk assessment or the prognosis of an outcome or the stratification of patients with nonspecific complaints based on the marker levels of proANP, proBNP, proAVP, pro ADM, proET-1, PCT, or PRX-4. WO 2010/009965 A1 relates to a method for risk stratification and/or prognosis and/or therapy control and/or monitoring of a patient having a cardiological disease or condition, comprising determining the level of a protease from azurophilic granules of polymorphonuclear neutrophils in a sample of the patient. EP 1 619 505 A2 relates to an in vitro method to diagnose in a critically ill patient the possible development or the presence of a life-threatening endothelin deficiency comprising determining in vitro in a sample of a body fluid of the patient the concentration of a pro-adrenomedullin (pro-ADM) and pro-endothelin (pro-END).

In order to identify high risk patients with adverse or fatal event/outcomes (i), to help clinicians in early decisions after ICU admission (ii), to optimize clinical treatments and resources (iii) and finally to decrease ICU mortality (iv), knowledge about determinant parameters like short- and long-term outcomes are of high value (Mayr, Dunser et al. 2006). Up to now, severity scores are mostly used for risk and severity assessment as well as outcome prediction of critically ill patients. Predominant ICU scoring systems are the acute physiology and chronic health evaluation (APACHE) score, the simplified acute physiology score (SAPS) and the sequential organ failure assessment (SOFA) score, based on 17, 14 or 6 physiology or organ specific parameters, respectively (Bouch and Thompson 2008). Despite their ability to predict a patient's outcome, such scores also have various disadvantages. For example, each single parameter of the scores has to be assessed and evaluated. Therefore, the determination of the results of the scores is time consuming (≥1 day), which is particularly disadvantageous in ICU care where fast tests are highly appreciated. Furthermore, such scores are dependent on the subjectivity of every assessing clinician, need to be recalibrated frequently and rely on several parameters to be individually determined.

Accordingly, there is a need of simple, fast and objective criteria for the diagnosis, prognosis, risk assessment, and/or risk stratification of an adverse event in a subject, particularly in a critically ill subject.

Therefore, the technical problem underlying the invention is the provision of means and methods to provide a fast and reliable way to predict an adverse event, particularly a fatal event, e.g. mortality/death, of a subject.

The technical problem is solved by provision of the embodiments provided herein below and as characterized in the appended claims.

### Description of the invention

The invention relates to a method for the prognosis of mortality of a subject, wherein said subject is a critically ill patient, and wherein said method comprises
(i) determining a level of at least one histone in a blood, blood plasma, or blood serum sample of said subject; and
(ii) determining a level of proadrenomedullin (proADM) in a in a blood, blood plasma, or blood serum sample of said subject; wherein

(i1) said level of at least one histone is compared to a reference level of at least one histone; and
(ii1) said level of proADM is compared to a reference level of proADM; and
(iii) wherein said mortality of said subject is identified based on the comparison in step (i1) and (ii1), and wherein said level of at least one histone and said level of proADM are indicative of said mortality occurring within 7 days.

The present invention solves the above identified technical problem. As documented herein below and in the appended examples, it was unexpectedly found in a clinical study that the levels of at least one histone, particularly histone H2B, H4, H2A and H3, and the level of proADM, particularly MR-proADM, demonstrate(s) a strong statistical relationship with mortality of the subjects; see illustrative Example 1. Accordingly, it is documented herein that said at least one histone protein and said proADM, e.g. MR-proADM, can be used as a marker for the prediction of mortality. In particular, the examples document that such markers can be employed for the prediction of a survivor or non-survivor, i.e. mortality of the subject.

In the appended examples, it is also surprisingly demonstrated that the level of the at least one histone, particularly H2B, H4, H2A and H3, is strongly associated with the adverse event, i.e. mortality, of the subject. In particular, the appended examples demonstrate that the levels of the histones are statistically associated with the adverse event, i.e. mortality, occurring within about 28 days, within about 7 days or within about 3 days. The appended examples revealed that an increase of the level of the marker indicate that the adverse event, i.e. mortality, occurs in the subject; see appended Example 1, e.g. Table 1 to 6. Moreover, the levels of the histones demonstrated to be particularly correlated to short adverse events, e.g. occurring within 7 days or 3 days. Furthermore, the level of the at least one histone in a sample of subjects suffering from respiratory disease was found to be correlated with the adverse event, i.e. mortality, occurring within 7 days; see e.g. table 4. In addition, the level of the at least one histone in a sample of the subjects suffering from urinary tract infection was found to be correlated with the adverse event, i.e. mortality, occurring within 28 days; see e.g. table 5. Moreover, the level of the at least one histone in a sample of the subject suffering from malignancies was found to be correlated with the adverse event, i.e. mortality, occurring within 28 days; see e.g. table 6.

In addition, it is also surprisingly demonstrated in the appended examples that the level of proADM, i.e. the level of the fragment MR-proADM, is strongly correlated with the adverse event, i.e. mortality, of the subject. In particular, the appended examples demonstrate that the level of proADM is statistically associated with the adverse event, i.e. mortality, occurring within about 28 days, within about 7 days or within about 3 days; see e.g. Tables 2 and 3. Particularly, the appended examples document that the level of proADM are indicative of the adverse event, e.g. mortality, occurring within 28 days or occurring within 7 days; see e.g. Tables 2 and 3.

In addition, it is documented in the appended examples that the prediction is further improved if a combination of the levels of the markers is determined. In particular, the determination of the level of at least one histone in addition to the level of proADM further improved the prognosis of the adverse event, i.e. mortality; see tables 1 to 3. It is documented in the appended examples that the determination of a level of a further marker and/or parameter in addition to the level of at least one histone or of proADM further improves the prediction of the adverse event, i.e. mortality; see illustrative tables 1 to 3. For example, it is demonstrated in the appended examples that determining a clinical score also improves the prediction based on the level of proADM or the histones. Also, the determination of the level of a biomarker, such as aldolase B, improves the prediction based on the level of proADM or the histones. Accordingly, the invention also relates to a method comprising determining the level of a further marker and/or parameter, i.e. the use of marker panels.

The present invention has, inter alia, the following advantages over the conventional methods: the inventive methods and use of the kits are fast, objective, easy to use and precise for the prediction of an adverse event: The methods and the use of kits of the invention relate to markers that are easily measurable in routine in hospitals because the levels of histones and of proADM can be determined in routinely obtained blood samples or further biological fluids obtained from a subject. In addition, the determination of the levels of the histones or proADM is very fast. Therefore, the methods and the use of kits of the invention are suitable for a quick assessment, and diagnosis and prognosis of mortality. Accordingly, the quick determination also is suitable for a fast treatment decision avoiding or reducing the risk of occurrence of the adverse event. Furthermore, due to the simple outcome of a biomarker measurement as one specific value, there is no subjective bias of medical staff when using this method or the use of kits of the invention. The reproducibility is thus higher compared to subjective scoring for physiological parameters as, for example, employed in the SOFA score. The level of the histone or proADM can also be combined with further marker(s) and/or parameter(s), e.g. clinical scores, such as SOFA score, in order to further improve the prediction and to adapt the analysis to specific sensitivities and specificities for evaluating the overall status of critical ill patients.

Accordingly, the herein provided methods and use of kits may be advantageous in the prognosis of mortality/death of a subject.

As documented herein above and in the appended examples, the level of histones and the level of proADM were surprisingly found to correlate with mortality in subjects. Accordingly, the invention relates to methods and use of kits for the prognosis of mortality of a subject.

Further, described herein but not claimed are methods and kits for monitoring, therapy guidance and/or therapy control of subjects, wherein the level of the at least one histone and/or of proADM is indicative of the adverse event, particularly mortality. The definitions provided herein above and below also apply to such aspects.

As used herein, the term "determining the level of at least one histone" or the like refers to determining a level of a histone or a fragment thereof in a sample of the subject or determining a level of more than one histones or fragments thereof in the sample of the subject. Particularly, "determining the level of at least one histone" may refer to determining a level of a histone in the sample of the subject, wherein preferably the histone is selected from the group consisting of histone H2B, H4, H2A and H3. Particularly, the level of the histone H2B is determined. Further, "determining the level of at least one histone" may refer to determining a level of a histone in the sample of the subject, wherein particularly the level of the histone H4 is determined. Further, "determining the level of at least one histone" may refer to determining a level of two histones in the sample of the subject, wherein preferably the levels of the histones H2B and H4 are determined. Further, "determining the level of at least one histone" may refer to determining a level of three histones in the sample of the subject, wherein preferably the levels of the histones H2B, H4, and H2A are determined. Further, "determining the level of at least one histone" may refer to determining a level of four histones in the sample of the subject, wherein preferably the levels of the histones H2B, H4, H2A and H3 are determined.

Accordingly, in the context of the present invention, "determining the level of at least one histone" may refer to determining a level of histone H2B, a level of histone H4, a level of histone H2A and/or a level of histone H3.

In particular, the term "determining the level of at least one histone" or the like may refer to determining a level of a histone in the sample of the subject. Accordingly, the invention also relates to a method for the prognosis of mortality of a subject, wherein said method comprises determining a level of a histone or a fragment thereof in a sample of said subject and wherein said level of a histone or said fragment thereof is indicative of said mortality.

As used herein, "histone" or "histone protein", or "histones" or "histone proteins" refers to the canonical histone(s), such as H1, H2A, H2B, H3 or H4, as well as histone variant(s), such as H3.3, H2A.Z etc. or fragment(s) thereof. Histones form the octamer particle around which DNA is wrapped in order to assemble the chromatin structure (Luger, Nature. 1997 Sep 18; 389(6648):251-60). For example, the histone proteins H2A, H2B, H3, and H4 (two of each) form an octamer, which is wrapped by 165 base pairs of DNA to form the fundamental subunit of chromatin, the nucleosome. Histones are also detected outside the nucleus in multiple pathophysiological processes (WO 2009/061918). The presence of extracellular histones has been described in the blood of patients suffering from different etiologies involving inflammatory processes. Histone release from activated immune cells can be mediated by extracellular traps. Activated neutrophils, as an ultimate mechanism of controlling and clearing an infection, can release extracellular fibers, so called neutrophile extracellular traps (NETs) (Brinkmann V., et al. Science 2004; 303(5663): p. 1532-5). Other mechanisms by which histones may be released into a patient's blood stream include apoptosis, necrosis, pyroptosis or necroptosis of cells.

In particular, the at least one histone is selected from the group consisting of H2B, H4, H2A and H3. Accordingly, the level of the histone to be determined in the methods and use of kits of the invention is particularly a level of the histones(s) H2B, H4, H2A and/or H3. The sequences of the histones are known to the skilled person. Exemplary sequences of the histones are given in SEQ ID NOs: 1 to 4. The exemplary amino acid sequence of histone H4 is given in SEQ ID NO: 1. The exemplary amino acid sequence of histone H2A is given in SEQ ID NO: 2. The exemplary amino acid sequence of histone H3 is given in SEQ ID NO: 3. The exemplary amino acid sequence of histone H2B is given in SEQ ID NO: 4. Particularly, the at least one histone is selected from the group consisting of H2B, H4, H2A and H3. More particularly, the at least one histone is selected from the group consisting of H2B, H4 and H2A. More particularly, the at least one histone is H2B and H4. More particularly, the at least one histone is H2B or H4.

It is understood that "determining the level of at least one histone" or the like refers to determining the level of at least one histone or a fragment of the at least one histone in the sample. In particular, the level of the histone H2B, H4, H2A, and/or H4 is determined in the sample. Accordingly, the at least one histone determined in the sample can be a free histone or the at least one histone determined in the sample can occur and can be assembled in a macromolecular complex, for example, in the octamer, nucleosome and/or NETs. Therefore, the level of at least one histone in the sample can comprise the level of free histone protein and/or histone protein assembled in a macromolecular complex.

A level of a histone or a fragment thereof may be determined in the sample that is not assembled in a macromolecular complex, such as a nucleosome, octamer or a neutrophil extracellular trap (NET). Such histone(s) are herein referred to as "free histone(s)". Accordingly, the level of the at least one histone may particularly be a level of at least one free histone.

The level of such free histones can be determined by the detection of amino acid sequences or structural epitopes of histones that are not accessible in an assembled stoichiometric macromolecular complex, like a mono-nucleosome or an octamer. In such structures, particular regions of the histones are covered and are thus sterically inaccessible as shown for the neutrophil extracellular traps ("NETs"), (Brinkmann V., et al. Science 303(5663): p. 1532-5, 2004). In addition, in the octamer or nucleosome, regions of histones also participate in intramolecular interactions, such as between the individual histones. Accordingly, the region/peptide/epitope of the histone that is determined may determine whether the histone is a free histone or a histone that is assembled in a macromolecular complex. For example, in an immunoassay based method, the utilized antibodies may not detect histones, e.g. H4, when they are part of the octameric core of nucleosomes as the epitopes are structurally inaccessible. Herein below, regions/peptides/epitopes of the histone are exemplified that could be employed to determine a free histone. For example, regions/peptides/epitopes of the N-terminal or C-terminal tail of the histones can be employed to determine histones independent of whether they are assembled in the macromolecular complex or are free histones.

It is understood that the determination of histones may include post-translational modified histone proteins. Accordingly, the post-translational modifications can comprise deacetylation or acetylation, phosphorylation, methylation, ubiquitylation and citrullination of amino acids.

"Stoichiometric" in this context relates to intact complexes, e.g. a mononucleosome or an octamer. "Free histone proteins" can also comprise non-chromatin-bound histones. For example, "free histone proteins" may also comprise individual histone proteins or non-octameric histone complexes. Free histones may (e.g. transiently) be bound to individual histones, for instance, histones may form homo- or hetero-dimers. The free histones may also form homo- or hetero-tetramers. The homo- or heterotetramer may consist of four molecules of histones, e.g. H2A, H2B, H3 and/or H4. A typical heterotetramer is formed by two heterodimers, wherein each heterodimer consists of H3 and H4. It is also understood herein that a heterotetramer may be formed by H2A and H2B. It is also envisaged herein that a heterotetramer may be formed by one heterodimer consisting of H3 and H4, and one heterodimer consisting of H2A and H2B. Free histones are thus herein referred to as and can be monomeric, heterodimeric or tetrameric histone proteins, which are not assembled in a ("stoichiometric") macromolecular complex consisting of the histone octamer bound to nucleic acid, e.g. a nucleosome. In addition, free histones may also be bound to nucleic acids, and wherein said free histones are not assembled in a ("stoichiometric") macromolecular complex, e.g. an intact nucleosome. Preferably, the free histone(s) is/are essentially free of nucleic acids.

The fragment of the at least one histone can have any length, e.g. at least about 5, 10, 20, 30, 40, 50 or 100 amino acids, so long as the fragment allows the unambiguous determination of the level of the particular histone. The fragment of the at least one histone refers to an independent fragment of the histones, e.g. of the histones H2B, H4, H2A and H3. Various exemplary fragments of the histones are disclosed herein below that are suitable to determine the level of the histone in the sample of the subject. It is also herein understood that the level of the histones can be determined by determining a fragment spanning the N-terminal or C-terminal tail of the histones. In addition, the histone or the fragment thereof to be determined may also be modified, e.g. by post-translational modification. Exemplary post translational modifications can be acetylation, citrullination, deacetylation, methylation, demethylation, deimination, isomerization, phosphorylation and ubiquitination.

As used herein, the term "proadrenomedullin" or "proADM" refers to proadrenomedullin or a fragment thereof, particularly MR-proADM. It is understood that "determining the level of proADM" or the like refers to determining proADM or a fragment thereof. The fragment can have any length, e.g. at least about 5, 10, 20, 30, 40, 50 or 100 amino acids, so long as the fragment allows the unambiguous determination of the level of the proADM. In particular preferred aspects of the invention, "determining the level of proADM" refers to determining the level of midregional proadrenomedullin (MR-proADM). MR-proADM is a fragment of proADM. The peptide adrenomedullin (ADM) was discovered as a hypotensive peptide comprising 52 amino acids, which had been isolated from a human phenochromocytomeby (Kitamura et al., 1993). Adrenomedullin (ADM) is encoded as a precursor peptide comprising 185 amino acids ("preproadrenomedullin" or "pre-proADM"). An exemplary amino acid sequence of ADM is given in SEQ ID NO: 5. ADM comprises the positions 95-146 of the pre-proADM amino acid sequence and is a splice product thereof. "Proadrenomedullin" ("proADM") refers to pre-proADM without the signal sequence (amino acids 1 to 21), i.e. to amino acid residues 22 to 285 of pre-proADM. "Midregional proadrenomedullin" ("MR-proADM") refers to the amino acids 42-95 of pre-proADM. An exemplary amino acid sequence of MR-proADM is given in SEQ ID NO: 6. It is also envisaged herein that a peptide and fragment thereof of pre-proADM or MR-proADM can be used for the herein described methods. For example, the peptide or the fragment thereof can comprise the amino acids 22-41 of pre-proADM (PAMP peptide) or amino acids 95-146 of pre-proADM (mature adrenomedullin). A C-terminal fragment of proADM (amino acids 153 to 185 of preproADM) is called adrenotensin. Fragments of the proADM peptides or fragments of the MR-proADM can comprise, for example, at least about 5, 10, 20, 30 or more amino acids. Accordingly, the fragment of proADM may, for example, be selected from the group consisting of MR-proADM, PAMP, adrenotensin and mature adrenomedullin, preferably herein the fragment is MR-proADM.

It is also envisaged herein that polypeptides can be determined, which have a sequence identity to proADM or to the at least one histone. For example, as disclosed herein polypeptides can be determined that have at least 75%, 80%, 85%, 90%, 91%, 92%, 93%, 94%, 95%, 96%, 97%, 98% or 99% sequence identity to SEQ ID NO: 5 or 6, or respectively to SEQ ID NO: 1, SEQ ID NO: 2, SEQ ID NO: 3, or SEQ ID NO: 4, wherein the higher values of sequence identity are preferred. As used herein, the terms "sequence identity", "homology" or "percent homology" or "identical" or "percent identity" or "percentage identity" in the context of two or more amino acid sequences may refer to two or more sequences or subsequences that are the same, or that have a specified percentage of amino acids that are the same, when compared and aligned for maximum correspondence over the window of comparison (preferably over the full length), or over a designated region as measured using a sequence comparison algorithm as known in the art, or by manual alignment and visual inspection. Sequences having, for example, 70% to 90% or greater (preferably 95% or greater) sequence identity may be considered to be substantially identical. Such a definition also applies to the complement of a test sequence. Preferably, the described identity exists over a region that is at least about 10 to about 15 amino acids in length, more preferably, over a region that is at least about 20 to about 35 amino acids in length, most preferably, over the full length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

As used herein, the "level" of the marker refers to the quantity of the molecular entity of the marker in the sample. In other words, the concentration of the marker is determined in the sample. Accordingly, the concentration of proADM or a fragment thereof, preferably MR-proADM, and the concentration of the histone(s) H2B, H4, H3 and/or H2A or (a) fragment(s) thereof is determined in the sample of the subject.

As used herein, the term "level of at least one histone" refers to the quantity of the molecular entity of the at least histone, e.g. the quantity of H2B, H4, H2A and/or H3, or a fragment thereof in a sample that is obtained from the subject. In other words, the concentration of the at least one histone protein or the fragment thereof is determined in the sample.

As used herein, the term "level of the marker proadrenomedullin (proADM)" or the "level of the marker proadrenomedullin (proADM) or a fragment thereof" refers to the quantity of the molecular entity of the marker proadrenomedullin or fragments thereof in a sample that is obtained from a subject. In other words, the concentration of the marker is determined in the sample. Hence, the term "level of the marker midregional proadrenomedullin (MR-proADM)" refers to the quantity of the molecular entity of the marker midregional proadrenomedullin (MR-proADM) in the sample that is obtained from a subject. As described above, it is also envisaged herein that a fragment of proadrenomedullin (proADM), preferably MR-proADM, can be detected and quantified. Also, fragments of MR-proADM can be detected and quantified. Suitable methods to determine the level of proADM or a fragment thereof (preferably MR-proADM) or to determine the level of the at least one histone or a fragment thereof are described herein below.

The "adverse event" means a health condition/status of the subject that is or will be life threatening. Therefore, the adverse event refers to a critical deterioration of the health condition/status of the subject in comparison to an earlier health condition of the same subject, e.g. that is diagnosed and confirmed e.g. about 28 days, 14 days, 7 days, 3 days, 1 day, 12 hours, 5 hours, 1 hour or less before the deterioration; or in comparison to the health condition of subjects suffering from (a) similar disease(s) or medical condition(s), i.e. the progression of the disease(s) or medical disorder(s) is or becomes life threatening in the subject tested. The adverse event also can refer to a critical deterioration of the health condition/status of the subject in comparison to the health condition of healthy subjects. It is herein understood that a critical deterioration of the health condition/status, a life threatening condition/status of the subject or life threatening progression can mean that the subject is at risk to die, i.e. the fatal outcome of the subject is likely.

A critical deterioration, critical health condition/status, or life threatening health condition/status can also be assessed/diagnosed by clinical scores, e.g. the SOFA score. For example, a SOFA score above 14 indicates a very severe health status indicating a critical health status of the subject. A SOFA score between 0 and 6 indicates a less severe health status and a SOFA score of 7 to 14 indicates a severe health status. For example, the health condition of the subject may critically deteriorate due to organ dysfunction, multiple organ dysfunctions, (a) disease(s) or medical disorder(s), such as an infection. Accordingly, the term "adverse event" may refer to an organ dysfunction, multiple organ dysfunctions, a disease or medical condition, such as an infection, that is or will be life threatening. For example, organ dysfunction or multiple organ dysfunction can lead to such a critical deterioration and thus the term "adverse event" may also refer to organ dysfunction or multiple organ dysfunctions, particularly organ failure or multiple organ failure. For example, a disease or medical disorder or can lead to such a critical deterioration and thus the term "adverse event" may also refer to (a) disease(s) or medical disorder(s), wherein said disease(s) or medical disorder(s) could mean disease(s) or medical disorder(s) that are life threatening. The subject can also suffer from (a) disease(s) and/or medical disorder(s) and the adverse event in such a scenario is the condition when the progression of the disease(s) or medical disorder(s) become(s) life threatening, i.e. the adverse event may also refer to a life threatening progression of the disease or disorder. A disease or disorder can be selected from the group consisting of respiratory disease, urinary tract infection, an inflammatory response related to infective and non-infective etiologies, systemic inflammatory response syndrome (SIRS), sepsis, severe sepsis, septic shock, organ failure(s), cardiovascular disease, hematologic disease, disseminated coagulation, diabetes mellitus, malignancy, liver disease, renal disease and/or immunodepression. The disease can also be (an) infection(s).

According to the invention, the adverse event is mortality. In other words, the adverse event is the death of the subject in particular aspects of the invention. Accordingly, the term "adverse event" means "mortality", or "death". As used herein, "mortality" means that the subject dies, i.e. the fatal outcome. Accordingly, the methods and kits of the invention determine/predict whether the subject will die and/or whether the subject is at risk to die.

The general term "organ dysfunction" can also mean that more than one organ has a dysfunction, i.e. it can also relate to organ dysfunctions unless stated otherwise. The term "organ dysfunction" or "organ dysfunctions" relates to a condition in the subject where an organ or more than one organ do(es) not perform its/their normal function compared to an unaffected organ, such for example the organ(s) of at least one healthy subject. For example, the organ(s) can have a reduced activity or the organ(s) can be abnormally active in the subject with the organ dysfunction in comparison to (an) organ(s) of at least one healthy subject. Preferably, the organ(s) with the organ dysfunction(s) can have a reduction or an increase of activity of at least about 10%, 20%, 30%, 50%, 70%, 90%, 100% or 200% compared to unaffected organ(s), e.g. of at least one healthy subject. In particular, organ dysfunction(s) can result in organ failure(s). Accordingly, "organ dysfunction(s)" can preferably also refer to organ failure(s). "Organ failure(s)" refers to (an) organ dysfunction(s) to such a degree that normal homeostasis cannot be maintained, e.g. without external clinical intervention. "Organ failure(s)" may also refer to (an) organ dysfunction(s) to such a degree that normal homeostasis of the organ(s) cannot be maintained, e.g. without external clinical intervention. "Organ failure(s)" may also refer to (an) organ dysfunction(s) to such a degree that normal homeostasis of the subject cannot be maintained, e.g. without external clinical intervention. The organ dysfunction may be an organ failure or at least one organ failure. The general term "organ failure" can also mean that more than one organ has a failure, i.e. it can also relate to organ failures unless stated otherwise. It is herein understood that organ dysfunctions can also be referred to as multiple organ dysfunction. It is herein understood that organ failures can also be referred to as multiple organ failure. Exemplary organ dysfunctions or organ failures are circulatory shock, hematologic failure, liver failure, neurologic failure, renal failure, respiratory failure and metabolic acidosis. Accordingly, the organ dysfunction or the at least one dysfunction can preferably be selected from the group consisting of circulatory shock, hematologic failure, liver failure, neurologic failure, renal failure, respiratory failure and metabolic acidosis. It is herein understood that the subject can also have more than one organ dysfunctions or failures that are e.g. a combination of two, three, four organ dysfunctions selected from the group consisting of circulatory shock, hematologic failure, liver failure, neurologic failure, renal failure, respiratory failure and metabolic acidosis.

Particularly, the level of at least one histone and the level of proADM, particularly MR-proADM, is indicative of mortality occurring within 7 days.

As described herein, said level of MR-proADM of said subject is indicative of the adverse event, i.e. mortality, of said subject occurring within 7 days.

As described herein, the level of at least one histone is indicative of the adverse event, i.e. mortality, occurring within 7 days. Particularly, the level of H2B is indicative of the adverse event, i.e. mortality, occurring within 7 days.

Particularly, the level of H4 is indicative of the adverse event, i.e. mortality, occurring within about 7 days.

The term "indicative of said adverse event" means that the subject has or will likely experience/have/suffer from said adverse event, i.e. mortality. Therefore, the level of the at least one histone and the level of proADM of the subject indicate(s) the adverse event, i.e. mortality of the subject.

As used herein, the term "is compared to a reference level of at least one histone" or grammatical variants thereof means that the level of the at least one histone of the subject is compared to a reference level of the at least one histone. Thus, a level of the histone of the subject is compared to a corresponding reference level of the same histone. For example, the level of the histone H2B determined in the sample of the subject is compared to a reference level of histone H2B. This applies mutatis mutandis to the other histones. The reference level of at least one histone is particularly a level of histone H2B, a level of histone H4, a level of histone H2A and/or a level of histone H3.

As used herein, the term "is compared to a reference level of proADM" or grammatical variants thereof means that the level of the proADM of the subject is compared to a reference level of the proADM. If a level of (a) fragment(s) of the at least one histone and/or of the proADM is determined the reference level may also be a level of (the) corresponding fragment(s).

As used herein, the "reference level" may reflect a normal level of the corresponding marker that is indicative of no mortality in preferred aspects of the invention. Accordingly, such a reference level reflects a normal level of the corresponding marker that does not indicate a life threatening condition or particularly death/mortality of the subject. Accordingly, the reference level may be a level of at least one histone and/or a level of proADM of at least one reference subject, and wherein the reference subject(s) has/have no adverse event within about 28 days, preferably within about 14 days, more preferably within about 7 days or particularly preferably within about 3 days. The reference level may represent the level of the at least one histone and/or the level of proADM of a group of healthy subjects (e.g. a cohort). Accordingly, the reference level is preferably a level of the at least one histone and/or a level of proADM, particularly MR-proADM of healthy subjects. A healthy subject is a subject with no diagnosed (and confirmed) disease and/or disorder. The healthy subjects may preferably have normally functioning organs, i.e. no organ dysfunction(s) or no organ failure(s), and/or no diagnosed disease(s) or medical disorder(s) such as those as described above. Accordingly, the reference level(s) can be a level of at least one histone and/or a level of proADM that is determined in samples of healthy subjects. The reference subjects or healthy subjects are herein preferably defined as a group of subjects or a group of healthy subjects, e.g. a cohort of subjects. The healthy reference subjects have no organ dysfunction or no organ failure. Accordingly, the reference level may be a level of the at least one histone and/or a level of proADM of subjects having no organ dysfunction or no organ failure. Accordingly, the reference level may be a level indicating no organ dysfunction or no organ failure.

Further, the reference level may also be a level of at least one histone and/or a level of proADM of at least one reference subject, wherein said reference subject(s) suffer(s) from a disease and/or medical disorder, and wherein the progression of the disease or disorder is not life threatening. In other words, the reference level can also be a level of at least one histone and/or a level of proADM of at least one reference subject, wherein said reference subject(s) suffer(s) from a disease and/or medical disorder, and wherein the adverse event, particularly mortality, does not occur within 28 days, 7 days or 3 days.

Further, the reference level may also be a level of at least one histone and/or a level of proADM of at least one reference subject, wherein said reference subject(s) suffer(s) from a disease and/or medical disorder and an infection (such as sepsis or septic disorders), and wherein the adverse event, particularly mortality, does not occur within 28 days, 7 days or 3 days. In other words, the adverse event of the reference subject does not occur within 28 days, 7 days or 3 days. Particularly, the reference subject suffers from the same disease or medical condition/disorder as the subject to be tested.

Further, the reference level may also be a level of at least one histone and/or a level of proADM of at least one reference subject, wherein said reference subject(s) suffer(s) from a disease and/or medical disorder and not from an infection, and wherein the adverse event, particularly mortality, does not occur within 28 days, 7 days or 3 days.

Further, the reference level may also be a level of at least one histone and/or a level of proADM of at least one reference subject, and wherein said reference subject(s) suffer(s) from a disease and/or medical disorder including SIRS, wherein said subject(s) do(es) not suffer from an infection, and wherein the adverse event, particularly mortality, does not occur within 28 days, 7 days or 3 days.

As used herein, the at least one reference subject refers to more than one reference subject. Particularly, the at least one reference subject is a group or a cohort of reference subjects. As described herein below, means and methods are described to determine the levels of the markers e.g. in reference.

The reference level as used herein is typically a predetermined level, i.e. it has been determined in advance as a reference for later use at the point-of-care, e.g. ICU.

According to the invention, the adverse event is mortality. In these aspects, the reference level may also be level of at least one histone and/or a level of proADM of at least one reference subject, wherein the reference subject(s) do(es) not die within about 28 days, within about 14 days, within about 7 days or within about 3 days. Further, in these aspects, the reference level can also be a level of at least one histone and/or a level of proADM of at least one reference subject, wherein said reference subject(s) suffer(s) from a disease and/or medical disorder, and wherein the reference subject(s) do(es) not die within about 28 days, within about 14 days, within about 7 days or within about 3 days. Further, in these aspects, the reference level can also be a level of at least one histone and/or a level of proADM of at least one reference subject, wherein said reference subject(s) suffer(s) from a disease and/or medical disorder and an infection, and wherein the reference subject(s) do(es) not die within about 28 days, within about 14 days, within about 7 days or within about 3 days. Further, in these aspects, the reference level can also be a level of at least one histone and/or a level of proADM of at least one reference subject, wherein said reference subject(s) suffer(s) from a disease and/or medical disorder and not from an infection, and wherein the reference subject(s) do(es) not die within about 28 days, within about 14 days, within about 7 days or within about 3 days. Further, in these aspects, the reference level can also be a level of at least one histone and/or a level of proADM of at least one reference subject, and wherein said reference subject(s) suffer(s) from a disease and/or medical disorder including SIRS, wherein said subject(s) do(es) not suffer from an infection, and wherein the reference subject(s) do(es) not die within about 28 days, within about 14 days, within about 7 days or within about 3 days.

As documented herein, an increased level of at least one histone (or an increase in the level of at least one histone) and/or an increased level of proADM (or an increase in the level of proADM), particularly MR-proADM, as compared to the reference level is indicative of an adverse event, particularly mortality. In particular, the adverse event occurs within 7 days as described above. Accordingly, said level of the at least one histone and/or said level of proADM indicating said adverse event may be an increased level as compared to a reference level.

As used herein, "obtained from prior analysis" refers to a determination of the marker level in the sample of the same subject at a pervious time, e.g. 28 days, 7 days, 6 days, 5 days, 4 days, 3 days, 2 days or 1 day prior to the next analysis, and wherein in such aspects said previously determined level of the marker is considered as the reference level.

As used herein, the term "increase in the level of (the) marker" means that the level of the marker is increased, i.e. it refers to an increased level of the marker. Accordingly, the term "increase in the level of (the) marker" is used interchangeably herein with the term "increased level of (the) marker". An increased level of the marker or an increase in the level of the marker of the subject means that the level of the marker is at least about 15%, preferably at least about 20%, more preferably at least about 25%, or even more preferably at least about 30% higher than the reference level of the marker.

In the context of the invention, the term "increase in the level of at least one histone as compared to the reference level" or the like is used interchangeably herein with the term "increased level of the at least one histone of said subject as compared to said reference level" or the term "increased level of the at least one histone as compared to said reference level" or the like. Such terms mean that the level of the at least one histone, e.g. the level of H2B, the level of H4, the level of H2A and/or the level of H3, of the subject is at least about 15%, preferably at least about 20%, more preferably at least about 25%, more preferably at least about 30%, more preferably at least about 40%, more preferably at least about 50%, more preferably at least about 60%, more preferably at least about 70%, more preferably at least about 80%, more preferably at least about 90%, most preferably at least about 100% higher than the reference level of the at least one histone. In other words the increased level of said at least one histone (or the increase in the level) may be about twice as high as said reference level of at least one histone. An increase of the level of about twice further increases the risk of the adverse event, e.g. mortality. For example, a hazard ratio of 1.45 of H4 as documented in table 1 means that the risk of an adverse event is further increased by 45%. Therefore, such values are also an indication of a strong correlation and optimal markers for the diagnosis.

As used herein, the term "increase in the level of proADM as compared to the reference level" or the like is used interchangeably herein with the term "increased level of the proADM of said subject as compared to said reference level" or the term "increased level of the proADM as compared to said reference level" or the like. Such terms mean that the level of proADM, particularly the level of MR-proADM, of the subject is at least about 15%, preferably at least about 20%, more preferably at least about 25%, or even more preferably at least about 30%, more preferably at least about 40%, more preferably at least about 50%, more preferably at least about 60%, more preferably at least about 70%, more preferably at least about 80%, more preferably at least about 90%, most preferably at least about 100% higher than the reference level of proADM, particularly MR-proADM. In other words, the increased level of said proADM may be about twice as high as said reference level of proADM.

It is herein understood that the reference levels and the determined marker levels (i.e. the levels that are determined for the individual subject at the point-of-care, e.g. ICU) can vary depending on the assay/method by which the levels are determined. For example, the reference level and the determined marker level determined by mass spectrometry based methods can be different from respective levels determined by immunoassays. The appended examples demonstrate that the levels of the markers can be determined by several methods, e.g. immunoassays and mass spectrometry based methods. The reference levels can be optimized by statistical methods as exemplified in the appended examples, such as Cox regression analysis. Hazard ratios may also be calculated. For example and as exemplified in the appended examples, Hazard ratios higher than 0 (HR>0) indicate a worse prognosis, i.e. that the adverse event likely occurs, and Hazard rations smaller than 0 indicating a good prognosis, i.e. that the adverse event does likely not occur. Accordingly, the skilled person is aware how to determine reference levels. For example, the levels (including reference levels) can be determined by an immunoassay, e.g. by determining the level of at least one histone and/or the level of proADM, e.g. MR-proADM, in samples of subjects (or reference subjects).

The reference level of the at least one histone may be about 100 ng/ml, more preferably about 90 ng/ml, more preferably about 80 ng/ml, more preferably about 70 ng/ml, more preferably about 60 ng/ml, more preferably about 50 ng/ml, more preferably about 45 ng/ml, more preferably about 40 ng/ml, or most preferably about 35 ng/ml. The reference level of the at least one histone may be about 10 ng/ml, more preferably about 15 ng/ml, more preferably about 20 ng/ml, more preferably about 25 ng/ml, more preferably about 30 ng/ml, or most preferably about 35 ng/ml. The reference level of the at least one histone may be about 10 ng/ml to about 100 ng/ml, about 10 ng/ml to about 90 ng/ml, more preferably about 10 ng/ml to about 60 ng/ml, more preferably about 10 ng/ml to about 40 ng/ml, more preferably about 15 ng/ml to about 40 ng/ml, or most preferably about 20 ng/ml to about 40 ng/ml.

The reference level of proADM may be about 4 nmol/L, more preferably about 5 nmol/L, more preferably about 7 nmol/L, more preferably about 8 nmol/L, more preferably about 9 nmol/L or particular preferably about 6 nmol/L.

Moreover, the levels (including reference levels) can be determined by mass spectrometric based methods, such as methods determining the relative quantification or determining the absolute quantification of the protein or fragment thereof of interest.

Relative quantification "rSRM" may be achieved by:
1. Determining increased or decreased presence of the target protein by comparing the SRM (Selected reaction monitoring) signature peak area from a given target fragment peptide detected in the sample to the same SRM signature peak area of the target fragment peptide in at least a second, third, fourth or more biological samples.
2. Determining increased or decreased presence of target protein by comparing the SRM signature peak area from a given target peptide detected in the sample to SRM signature peak areas developed from fragment peptides from other proteins, in other samples derived from different and separate biological sources, where the SRM signature peak area comparison between the two samples for a peptide fragment are normalized for e.g. to amount of protein analyzed in each sample.
3. Determining increased or decreased presence of the target protein by comparing the SRM signature peak area for a given target peptide to the SRM signature peak areas from other fragment peptides derived from different proteins within the same biological sample in order to normalize changing levels of histones protein to levels of other proteins that do not change their levels of expression under various cellular conditions.
4. These assays can be applied to both unmodified fragment peptides and to modified fragment peptides of the target proteins, where the modifications include, but are not limited to phosphorylation and/or glycosylation, acetylation, methylation (mono, di, tri), citrullination, ubiquitinylation and where the relative levels of modified peptides are determined in the same manner as determining relative amounts of unmodified peptides.

Absolute quantification of a given peptide may be achieved by:
1. Comparing the SRM/MRM signature peak area for a given fragment peptide from the target proteins in an individual biological sample to the SRM/MRM signature peak area of an internal fragment peptide standard spiked into the protein lysate from the biological sample. The internal standard may be a labeled synthetic version of the fragment peptide from the target protein that is being interrogated or the labeled recombinant protein. This standard is spiked into a sample in known amounts before (mandatory for the recombinant protein) or after digestion, and the SRM/MRM signature peak area can be determined for both the internal fragment peptide standard and the native fragment peptide in the biological sample separately, followed by comparison of both peak areas. This can be applied to unmodified fragment peptides and modified fragment peptides, where the modifications include but are not limited to phosphorylation and/or glycosylation, acetylation, methylation (e.g. mono-, di-, or tri-methylation), citrullination, ubiquitinylation, and where the absolute levels of modified peptides can be determined in the same manner as determining absolute levels of unmodified peptides.
2. Peptides can also be quantified using external calibration curves. The normal curve approach uses a constant amount of a heavy peptide as an internal standard and a varying amount of light synthetic peptide spiked into the sample. A representative matrix similar to that of the test samples needs to be used to construct standard curves to account for a matrix effect. Besides, reverse curve method circumvents the issue of endogenous analyte in the matrix, where a constant amount of light peptide is spiked on top of the endogenous analyte to create an internal standard and varying amounts of heavy peptide are spiked to create a set of concentration standards. Test samples to be compared with either the normal or reverse curves are spiked with the same amount of standard peptide as the internal standard spiked into the matrix used to create the calibration curve.

Accordingly, the skilled person is aware how to determine marker levels and in particular appropriate reference levels as is also exemplified in the appended examples.

The sensitivity and specificity of the provided methods depend on more than just the analytical quality of the test. Sensitivity and specificity also depend on the definition of what constitutes an abnormal (e.g. mortality) or normal result. The distribution of levels of the at least one histone and/or of proADM, preferably the level of MR-proADM, for subjects with and without the adverse event may overlap. Under such conditions, a test does not absolutely distinguish normal from a dysfunctioning state with 100% accuracy. The skilled person is aware of the fact that the condition per se of a subject or at least one further maker and/or parameter of the subject can assist in the interpretation of the data and that this further information allows a more reliable prognosis in the areas of overlap. Accordingly, the level(s) of at least one further marker and/or parameter (e.g. sex, group and age) is determined. The levels of at least one further marker and/or parameter can also be compared to reference levels, wherein similar or identical values/levels of said at least one further marker and/or parameter of the subject compared to the corresponding levels of said at least one further marker and/or parameter of said reference levels indicate that the risk of the subject to experience/have/suffer from an adverse event is decreased, and/or wherein higher or lower levels/values of said at least one further marker and/or parameter compared to the corresponding levels of said at least one further marker and/or parameter of said reference levels indicate that the risk to experience/have/suffer from an adverse event is increased.

Accordingly, the methods and use of kits of the present invention can also comprise determining at least one further marker and/or parameter in addition to the at least one histone and proADM.

As used herein, a parameter is a characteristic, feature, or measurable factor that can help in defining a particular system. A parameter is an important element for health- and physiology-related assessments, such as a disease/disorder/clinical condition risk, preferably an adverse event. Furthermore, a parameter is defined as a characteristic that is objectively measured and evaluated as an indicator of normal biological processes, pathogenic processes, or pharmacologic responses to a therapeutic intervention. An exemplary parameter can be selected from the group consisting of Acute Physiology and Chronic Health Evaluation score (APACHE scores I-IV), the simplified acute physiology score (SAPS I-III score), sequential organ failure assessment score (SOFA score), simplified acute physiology score II (SAPSII score) , mortality probability model (MPM I-III), multiple organ dysfunction score (MODS), therapeutic intervention scoring system (TISS), nine equivalents of nursing manpower use score (NEMS), World Federation of Neurosurgical Societies (WFNS) grading, and Glasgow Coma Scale (GCS), age, gender, family history, ethnicity, body weight, body mass index (BMI), cystoscopy report, white blood cell count, CT scan, blood pressure, heart rate, antihypertensive treatment, liquid intake, wheezing, body temperature, presence of diabetes mellitus and current smoking habits.

As used herein, terms such as "marker", "surrogate", "prognostic marker", "factor" or "biomarker" or "biological marker" are used interchangeably and relate to measurable and quantifiable biological markers (e.g., specific protein or enzyme concentration or a fragment thereof, specific hormone concentration or a fragment thereof, or presence of biological substances or a fragment thereof) which serve as indices for health- and physiology-related assessments, such as a disease/disorder/clinical condition risk, preferably an adverse event. A marker is defined as a characteristic that can be objectively measured and evaluated as an indicator of normal biological processes, pathogenic processes, or pharmacologic responses to a therapeutic intervention. Biomarkers may be measured in a sample (as a blood, plasma, urine, or tissue test). The at least one further marker of said subject can be selected from the group consisting of procalcitonin, calcitonin, Endothelin-1 (ET-1), Arginine Vasopressin (AVP), Atrial Natriuretic Peptide (ANP), Neutrophil Gelatinase-Associated Lipocalin (NGAL), Troponin, Brain Natriuretic Peptide (BNP), C-Reactive Protein (CRP), Pancreatic Stone Protein (PSP), Triggering Receptor Expressed on Myeloid Cells 1 (TREM1), Interleukin-6 (IL-6), Interleukin-1, Interleukin-24 (IL-24) other ILs, Presepsin (sCD14-ST), Lipopolysaccharide Binding Protein (LBP), Alpha-1-Antitrypsin, Matrix Metalloproteinase 2 (MMP2), Matrix Metalloproteinase 9 (MMP9), Matrix Metalloproteinase 7 (MMP9), Chromogranin A, S100A protein, S100B protein and Tumor Necrosis Factor α (TNFα) or a fragment thereof.

In particular aspects of the invention, the at least one further marker and/or parameter of said subject can be selected from the group consisting of a level of aldolase B in said sample, a level of copeptin in said sample, a level of lactate in said sample, a level of procalcitonin (PCT) in said sample, the sequential organ failure assessment score (SOFA score) of said subject, the simplified acute physiology score (SAPSII score), the Acute Physiology and Chronic Health Evaluation II (APACHE II) score of said subject and a level of the soluble fms-like tyrosine kinase-1 (sFlt-1) in said sample.

As disclosed herein the at least one further marker and/or parameter of said subject may be selected from the group consisting of procalcitonin, calcitonin, Endothelin-1 (ET-1), Arginine Vasopressin (AVP), Atrial Natriuretic Peptide (ANP), Neutrophil Gelatinase-Associated Lipocalin (NGAL), Troponin, Brain Natriuretic Peptide (BNP), C-Reactive Protein (CRP), Pancreatic Stone Protein (PSP), Triggering Receptor Expressed on Myeloid Cells 1 (TREM1), Interleukin-6 (IL-6), Interleukin-1, Interleukin-24 (IL-24) other ILs, Presepsin (sCD14-ST), Lipopolysaccharide Binding Protein (LBP), Alpha-1-Antitrypsin, Matrix Metalloproteinase 2 (MMP2), Matrix Metalloproteinase 9 (MMP9), Matrix Metalloproteinase 7 (MMP7), Chromogranin A, S100A protein, S100B protein, Tumor Necrosis Factor α (TNFα), age, gender, family history, ethnicity, body weight, body mass index (BMI), cystoscopy report, white blood cell count, CT scan, blood pressure, heart rate, antihypertensive treatment, liquid intake, wheezing, , body temperature, presence of diabetes mellitus, current smoking habits, Acute Physiology and Chronic Health Evaluation score (APACHE scores I-IV), the simplified acute physiology score (SAPS I-III score), sequential organ failure assessment score (SOFA score), mortality probability model (MPM I-III), multiple organ dysfunction score (MODS), therapeutic intervention scoring system (TISS), nine equivalents of nursing manpower use score (NEMS), World Federation of Neurosurgical Societies (WFNS) grading, and Glasgow Coma Scale (GCS).

As used herein, "Aldolase B" refers to fructose-bisphosphate aldolase B or liver-type aldolase that is one of three isoenzymes (A, B, and C) of the class I fructose 1,6-bisphosphate aldolase enzyme. The level of Aldolase B in the sample of the subject can be determined by mass spectrometry based methods.

"Copeptin" is also referred to as "CT-proAVP" or "C-terminal portion of vasopressin". Vasopressin is a powerful vasoconstrictor. The level of CT-proAVP can be measured in the plasma or serum of a subject by immunoassays as described below.

As used herein, "lactate" refers to the maximal lactate concentration measured in the blood. Normally, the lactate concentration is assessed daily or even more often. The lactate concentration in the blood can be determined by lactate oxidase spectrophotometric methods.

As used herein, "procalcitonin" or "PCT" relates to a peptide spanning amino acid residues 1-116, 2-116, 3-116 or fragments thereof. Thus the length of procalcitonin fragments is at least 12 amino acids, preferably more than 50 amino acids, more preferably more than 110 amino acids. PCT may comprise post-translational modifications such as glycosylation, liposidation or derivatisation. Procalcitonin is a precursor of calcitonin and katacalcin. Thus, under normal conditions the PCT levels in the circulation are very low (< about 0.05 ng/ml). The level of PCT in the sample of the subject can be determined by immunoassays as described below.

As used herein, the "sequential organ failure assessment score" or "SOFA score" is one score used to track a patient's status during the stay in an intensive care unit (ICU). The SOFA score is a scoring system to determine the extent of a person's organ function or rate of failure. The score is based on six different scores, one each for the respiratory, cardiovascular, hepatic, coagulation, renal and neurological systems. Both the mean and highest SOFA scores being predictors of outcome. An increase in SOFA score during the first 24 to 48 hours in the ICU predicts a mortality rate of at least 50% up to 95%. Scores less than 9 give predictive mortality at 33% while above 14 can be close to or above 95%.

As used herein, "SAPS II" or "Simplified Acute Physiology Score II" relates to a system for classifying the severity of a disease or disorder (see Le Gall JR et al., A new Simplified Acute Physiology Score (SAPS II) based on a European/North American multicenter study. JAMA. 1993;270(24):2957-63.). The SAPS II score is made of 12 physiological variables and 3 disease-related variables. The point score is calculated from 12 routine physiological measurements, information about previous health status and some information obtained at admission to the ICU. The SAPS II score can be determined at any time, preferably, at day 2. The "worst" measurement is defined as the measure that correlates to the highest number of points. The SAPS II score ranges from 0 to 163 points. The classification system includes the followings parameters: Age, Heart Rate, Systolic Blood Pressure, Temperature, Glasgow Coma Scale, Mechanical Ventilation or CPAP, PaO2, FiO2, Urine Output, Blood Urea Nitrogen, Sodium, Potassium, Bicarbonate, Bilirubin, White Blood Cell, Chronic diseases and Type of admission. There is a sigmoidal relationship between mortality and the total SAPS II score. The mortality of a subject is 10% at a SAPSII score of 29 points, the mortality is 25% at a SAPSII score of 40 points, the mortality is 50% at a SAPSII score of 52 points, the mortality is 75% at a SAPSII score of 64 points, the mortality is 90% at a SAPSII score of 77 points (Le Gall loc. cit.).

As used herein, "APACHE II" or "Acute Physiology and Chronic Health Evaluation II" is a severity-of-disease classification scoring system (Knaus et al., 1985). It can be applied within 24 hours of admission of a patient to an intensive care unit (ICU) and may be determined based on 12 different physiologic parameters.

As used herein, "Soluble fms-like tyrosine kinase-1" or "sFlt-1" is a tyrosine kinase protein that disables proteins that cause blood vessel growth. Soluble Flt-1 (sFlt-1) is a splice variant of VEGF receptor 1 (Flt-1) which is produced by a variety of tissues. The level of sFLT1 in the sample of the subject can be determined by mass spectrometry based assays and immunoassays.

The methods or the use of kits of the invention can comprise determining the level of at least one histone and the level of proADM and a level of a further marker and/or parameter as described above.

The methods and the employed kits of the present invention may also comprise determining at least one further parameter, such as the SAPS II score, SOFA score and/or APACHE II score. For example, the method can comprise determining the level of at least one histone and/or the level of proADM in the sample of the subject and the SAPS II score of the subject. Preferably, the method comprises determining said level of at least one histone in said sample of said subject and said SAPSII score of said subject. The level of at least one histone and the SAPSII score can be indicative of the adverse event, particularly mortality, of said subject occurring within 28 days. Further, the method may comprise determining the level of at least one histone and the level of proADM in the sample of the subject and the SOFA score of the subject.

Further, the method can comprise determining the level of at least one histone and the level of proADM in the sample of the subject and the level of PCT in the sample of the subject. Further, the method can comprise determining the level of at least one histone and the level of proADM in the sample of the subject and the level of aldolase B in the sample of the subject.

As used herein, the "subject" (or "patient") may be a vertebrate. As used herein, the term "subject" may include both humans and animals, particularly mammals, and other organisms. Thus, the herein provided methods are applicable to both human and animal subjects. Accordingly, said subject may be an animal such as a mouse, rat, hamster, rabbit, guinea pig, ferret, cat, dog, chicken, sheep, bovine species, horse, camel, or primate. Preferably, the subject is a mammal. Most preferably, the subject is human.

The method provided herein can be used on any subject that is a healthy subject or a subject that suffers from any diseases(s) or disorder(s). According to the invention, the subject to be tested is a critical ill patient, preferably wherein said subject is admitted to an intensive care unit. As used herein, critical ill means that the subject or patient is in a life threatening situation.

The subject or the reference subject(s) may suffer from a disease or medical condition/disorder and wherein said disease or medical condition/disorder may be selected from the group consisting of respiratory disease, urinary tract infection, an inflammatory response related to infective and non-infective etiologies, systemic inflammatory response syndrome (SIRS), sepsis, severe sepsis, septic shock, organ failure(s), cardiovascular disease, diabetes mellitus, malignancy(ies), liver disease, renal disease and/or immunodepression.

Particularly, the subject of suffers from respiratory disease, urinary tract infection, and/or malignancy(ies).

"Systematic inflammation" relates to a condition characterized by a release of pro-inflammatory cytokines and an activated innate immune system which can be caused by biological factors, chemical factors or by genetic factors. Severe "Systemic Inflammation" can lead to organ failure and death.

"SIRS" in the context of the invention is a systemic inflammatory response syndrome with no signs of infection. It includes, but is not limited to more than one of the following clinical manifestations: (1) a body temperature greater than 38°C or less than 36°C; (2) a heart rate greater than 90 beats per minute; (3) tachypnea, manifested by a respiratory rate greater than 20 breaths per minute, or hyperventilation, as indicated by a PaCO₂ of less than 32 mm Hg; and (4) an alteration in the white blood cell count such as a count greater than 12,000/mm³, a count less than 4,000/mm³, or the presence of more than 10% immature neutrophiles (Bone et al., CHEST 101(6): 1644-55, 1992).

"Sepsis" in the context of the invention refers to a systemic response to infection. Alternatively, sepsis may be seen as the combination of SIRS with a confirmed infectious process or an infection. Sepsis may be characterized as clinical syndrome defined by the presence of both infection and a systemic inflammatory response (Levy MM et al. 2001 SCCM/ESICM/ACCP/ATS/SIS International Sepsis Definitions Conference. Crit Care Med. 2003 Apr;31(4):1250-6). The term "sepsis" used herein includes, but is not limited to, sepsis, severe sepsis, septic shock. Severe sepsis in this context means sepsis associated with organ dysfunction, hypoperfusion abnormality, or sepsis-induced hypotension. Hypoperfusion abnormalities include lactic acidosis, oliguria and acute alteration of mental status. Sepsis-induced hypotension is defined by the presence of a systolic blood pressure of less than about 90 mm Hg or its reduction by about 40 mm Hg or more from baseline in the absence of other causes for hypotension (e.g. cardiogenic shock). Septic shock is defined as severe sepsis with sepsis-induced hypotension persisting despite adequate fluid resuscitation, along with the presence of hypoperfusion abnormalities or organ dysfunction (Bone et al., CHEST 101(6): 1644-55, 1992).

The term "sepsis" used herein relates to all possible stages in the development of sepsis.

As used herein, "infection" within the scope of the invention means a pathological process caused by the invasion of normally sterile tissue or fluid by pathogenic or potentially pathogenic microorganisms and relates to infection(s) by bacteria, viruses, fungi, and/or parasites. Accordingly, the infection can be a bacterial infection, viral infection, and/or fungal infection. The infection can be a local or systemic infection. Further, the subject suffering from an infection can suffer from more than one source(s) of infection simultaneously. For example, the subject suffering from an infection can suffer from a bacterial infection and viral infection; from a viral infection and fungal infection; from a bacterial and fungal infection, and from a bacterial infection, fungal infection and viral infection.

Particularly, the subject suffers from sepsis. Further, the subject may preferably suffer from a respiratory disease, preferably an infection of the lower respiratory tract. As used herein respiratory disease comprises pathological conditions affecting the organs and tissues that make gas exchange possible in higher organisms, and also includes conditions of the upper respiratory tract, trachea, bronchi, bronchioles, alveoli, pleura and pleural cavity, and the nerves and muscles of breathing.

As used herein, the term "sample" is a biological sample that is obtained from the subject. "Sample" as used herein may, e.g., refer to a sample of bodily fluid or tissue obtained for the purpose of diagnosis, prognosis, or evaluation of a subject of interest, such as a patient. The samples could be processed (pre-treated), such as by fractionation or purification procedures, for example, separation of whole blood into serum or plasma components. Such pre-treatments can also include, but are not limited to dilution, filtration, centrifugation, concentration, sedimentation, precipitation or dialysis. Pre-treatments may also include the addition of chemical or biochemical substances to the solution, such as acids, bases, buffers, salts, solvents, reactive dyes, detergents, emulsifiers, chelators. According to the invention, the sample is a blood sample, more preferably a serum sample or a plasma sample.

"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood), for example for at least 15 minutes at 2000 to 3000 g.

"Serum" in the context of the present invention is the liquid fraction of whole blood that is collected after the blood is allowed to clot. When coagulated blood (clotted blood) is centrifuged serum can be obtained as supernatant.

As used herein, "urine" is a liquid product of the body secreted by the kidneys through a process called urination (or micturition) and excreted through the urethra.

As described above, the level of at least one histone and of proADM is determined in the sample of the subject. The skilled person is aware of methods/assay that can be employed to determine the level of biomarkers in a sample.

As described above, the level of at least one histone is determined, particularly, the level(s) of the histones H2B, H4, H2A and/or H3 is/are determined. Particularly, the histone or the histone fragment can be determined. Such fragments are herein exemplified below.

Such a histone or fragment thereof may also represent a free histone. For example, the methods and use of kits of the present invention may particularly detect peptides or epitopes of free histones. Such stretches of amino acids are also referred herein as central regions or parts of the histones. In the following, peptides or epitopes are described that may also be employed to detect free histones using the methods herein provided.

In particular, the at least one histone can be histone H4 and wherein at least a peptide of the sequence spanning amino acid residues 22 to 102 of histone H4 according to SEQ ID NO:1 is determined. Particularly, the least one histone is histone H4 and wherein at least a peptide of the sequence is determined selected from the group consisting of an amino acid sequence spanning residues 60 to 67 of SEQ ID NO: 1, residues 46 to 56 of SEQ ID NO:1, residues 67 to 78 of SEQ ID NO: 1, residues 22 to 30 of SEQ ID NO: 1, residues 67 to 78 of SEQ ID NO: 1, residues 92 to 102 of SEQ ID NO: 1, residues 22 to 34 of SEQ ID NO: 1, residues 46 to 102 of SEQ ID NO: 1, residues 46 to 55 of SEQ ID NO: 1, residues 80 to 91 of SEQ ID NO: 1, residues 24 to 35 of SEQ ID NO: 1, and residues 68 to 77 of SEQ ID NO: 1. For example, two peptides may be determined Particularly, the least one histone is histone H4 and wherein the peptides are determined of an amino acid sequence spanning residues 46 to 56 of SEQ ID NO:1 and residues 67 to 78 of SEQ ID NO: 1.

Further, the least one histone is histone H2A and wherein at least a peptide of the sequence spanning amino acid residues 20 to 118 of histone H2A according to SEQ ID NO: 2 is determined. In particular, the least one histone is histone H2A and wherein at least a peptide of the sequence is determined selected from the group consisting of an amino acid sequence spanning residues 21 to 53 of SEQ ID NO: 2, residues 21 to 29 of SEQ ID NO: 2, residues 30 to 53 of SEQ ID NO: 2, residues 120 to 129 of SEQ ID NO: 2, residues 21 to 29 of SEQ ID NO: 2, residues 82 to 88 of SEQ ID NO: 2, residues 89 to 95 of SEQ ID NO: 2, and residues 100 to 118 of SEQ ID NO: 2.

Further, the least one histone is histone is histone H3 and wherein at least a peptide of the sequence spanning amino acid residues 27 to 62 of histone H3 according to SEQ ID NO: 3 is determined. Further, the least one histone is histone H3 and wherein at least a peptide of the sequence spanning amino acid residues 27 to 37 of SEQ ID NO: 3 and/or spanning amino acid residues 52 to 62 of SEQ ID NO: 3 is determined.

Further, the least one histone is histone H2B and wherein at least a peptide of the sequence spanning amino acid residues 41 to 69 of histone H2B according to SEQ ID NO: 4 is determined.

Further, the least one histone is histone H2A and wherein at least a peptide or a fragment thereof is determined selected from the group consisting of SEQ ID NOs: 7, 8, 9 and 10 is determined.

Further, the least one histone is histone H4 and wherein at least a peptide or a fragment thereof selected from the group consisting of SEQ ID NOs: 11, 12, 13, 14, 15 and 16 is determined. It is herein understood that one, two three, four or more peptides can be determined.

The level of the markers, e.g. the at least one histone or the fragment thereof and/or the proADM or the fragment thereof, can be determined by any assay that reliably determines the concentration of the marker. Particularly, mass spectrometry (MS) and/or immunoassays can be employed as exemplified in the appended examples. As used herein, an immunoassay is a biochemical test that measures the presence or concentration of a macromolecule/polypeptide in a solution through the use of an antibody or antibody binding fragment or immunoglobulin.

Alternatively, instead of antibodies, other capture molecules or molecular scaffolds that specifically and/or selectively recognize histone sequences, histone epitopes, and structural conformations of histones may be used. Herein, the term "capture molecules" or "molecular scaffolds" comprises molecules which may be used to bind target molecules or molecules of interest, i.e. analytes (e.g. the histone(s) and/or proADM), from a sample. Capture molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may, for instance, be mediated by ionic, van-der-Waals, pi-pi, sigma-pi, hydrophobic or hydrogen bond interactions or a combination of two or more of the aforementioned interactions or covalent interactions between the capture molecules or molecular scaffold and the target molecules or molecules of interest. Capture molecules or molecular scaffolds may for instance be selected from the group consisting of a nucleic acid molecule, a carbohydrate molecule, a PNA molecule, a protein, a peptide and a glycoprotein. Capture molecules or molecular scaffolds include, for example, aptamers, DARpins (Designed Ankyrin Repeat Proteins), Affimers and the like.

As used herein, the term, "antibody" refers to immunoglobulin molecules and immunologically active portions of immunoglobulin (Ig) molecules, i.e., molecules that contain an antigen binding site that specifically binds (immuno reacts with) an antigen. The antibodies may be monoclonal as well as polyclonal antibodies. Particularly, antibodies that are specifically binding to the at least one histone and/or that bind specifically to proADM are used. An antibody is considered to be specific, if its affinity towards the molecule of interest, e.g. the at least one histone and/or proADM, or the fragment thereof is at least 50-fold higher, preferably 100-fold higher, most preferably at least 1000-fold higher than towards other molecules comprised in a sample containing the molecule of interest. It is well known in the art how to develop and to select antibodies with a given specificity. Monoclonal antibodies may be preferred. The antibody or the antibody binding fragment binds specifically to the herein defined markers or fragments thereof. In particular, the antibody or the antibody binding fragment binds to the herein defined peptides of the at least one histone protein. Thus, the herein defined peptides can also be epitopes to which the antibodies specifically bind to. Further, an antibody or an antibody binding fragment may be used in the methods and kits of the invention as defined in the claims that binds specifically to proADM, particularly to MR-proADM. Exemplary immunoassays can be luminescence immunoassay (LIA), radioimmunoassay (RIA), chemiluminescence- and fluorescence- immunoassays, enzyme immunoassay (EIA), Enzyme-linked immunoassays (ELISA), luminescence-based bead arrays, magnetic beads based arrays, protein microarray assays, rapid test formats, rare cryptate assay. Further, assays suitable for point-of-care testing and rapid test formats such as for instance immune-chromatographic strip tests can be employed.

In certain aspects of the invention as defined in the claims, the method for the prognosis of mortality of a subject comprises
(i) obtaining a sample of the subject;
(ii) detecting a level of at least one histone and a level of proadrenomedullin (proADM) in the sample of said subject by contacting the sample with antibodyies or antigen-binding fragments or derivatives thereof specific for a epitope of said at least one histone and of said proADM and detecting binding between the antibodyies or the antigen-binding fragments or derivatives thereof and said at least one histone and said proADM; and
(iii) wherein said level of at least one histone and said level of proadrenomedullin (proADM) are indicative of is indicative of said adverse event of said subject.

In certain aspects of the invention as defined in the claims, the method is an immunoassay comprising the steps of:
a) contacting the sample with
   (i) a first antibody or an antigen-binding fragment or derivative thereof specific for a first epitope of a histone or of proADM, and
   (ii) a second antibody or an antigen-binding fragment or derivative thereof specific for a second epitope of the histone or the proADM; and
b) detecting the binding of the first and second antibodies or antigen-binding fragments or derivates thereof to the histone or to proADM.

Preferably, one of the antibodies can be labeled and the other antibody can be bound to a solid phase or can be bound selectively to a solid phase. In a particularly preferred aspect of the assay, one of the antibodies is labeled while the other is either bound to a solid phase or can be bound selectively to a solid phase. The first antibody and the second antibody can be present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to said at least one histone and/or to said proADM to be detected, a measurable signal which permits detection of the resulting sandwich complexes in the measuring solution is generated. The labelling system can comprise a rare earth cryptate or chelate in combination with a fluorescent or chemiluminescent dye, in particular of the cyanine type.

The method may be executed as heterogeneous sandwich immunoassay, wherein one of the antibodies is immobilized on an arbitrarily chosen solid phase, for example, the walls of coated test tubes (e.g. polystyrol test tubes; coated tubes; CT) or microtiter plates, for example composed of polystyrol, or to particles, such as for instance magnetic particles, whereby the other antibody has a group resembling a detectable label or enabling for selective attachment to a label, and which serves the detection of the formed sandwich structures. A temporarily delayed or subsequent immobilization using suitable solid phases is also possible.

The method as disclosed herein may furthermore be embodied as a homogeneous method, wherein the sandwich complexes formed by the antibody/antibodies and the marker, e.g., the histone or the proADM or a fragment thereof, which is to be detected remains suspended in the liquid phase. In this case it is preferred, that when two antibodies are used, both antibodies are labeled with parts of a detection system, which leads to generation of a signal or triggering of a signal if both antibodies are integrated into a single sandwich. Such techniques are to be embodied in particular as fluorescence enhancing or fluorescence quenching detection methods. A particularly preferred aspect relates to the use of detection reagents which are to be used pair-wise, such as for example the ones which are described in US 4 882 733 A, EP-B1 0 180 492 or EP-B1 0 539 477 and the prior art cited therein. In this way, measurements in which only reaction products comprising both labeling components in a single immune-complex directly in the reaction mixture are detected, become possible. For example, such technologies are offered under the brand names TRACE^{®} (Time Resolved Amplified Cryptate Emission) or KRYPTOR^{®}, implementing the teachings of the above-cited applications. Therefore, in particular preferred aspects, a diagnostic device is used to carry out the herein provided method. For example, the level of the histone or proADM or a fragment thereof, and/or the level of any further marker of the herein provided method is determined. In particular preferred aspects, the diagnostic device is KRYPTOR^{®}.

Further, the immunoassay methods of the present invention as defined in the claims may preferably utilize a first antibody and/or a second antibody or antigen-binding fragment(s) or derivative(s) thereof being specific for (an) epitope(s) of at least one histone and/or of proADM. Exemplary, peptides are described herein below and above that can be suitable for the determination of the level of proADM and/or of the at least one histone.

For example, the immunoassay methods of the present invention as defined in the claims may preferably utilize a first antibody and/or a second antibody or antigen-binding fragment(s) or derivative(s) thereof being specific for (an) epitope(s) of histone H4, wherein the first epitope and/or second epitope are epitopes of histone H4 present in the sequence spanning amino acid residues 22 to 102 of SEQ ID NO:1.

Further, the immunoassay methods of the present invention as defined in the claims may utilize a first antibody, antigen-binding fragment or derivative thereof that is specific for an epitope of histone H4 present in the sequence spanning amino acid residues 46 to 56 of SEQ ID NO:1, and a second antibody, antigen-binding fragment or derivative thereof that is specific for an epitope of histone H4 present in the sequence spanning amino acid residues 67 to 78 of SEQ ID NO: 1.

For example, the immunoassay methods of the present invention as defined in the claims may preferably utilize a first antibody and/or a second antibody or antigen-binding fragment(s) or derivative(s) thereof being specific for (an) epitope(s) of histone H2B, wherein the first epitope and/or second epitope are epitopes of histone H2B present in the sequence spanning amino acid residues 41 to 69 of SEQ ID NO:4.

Further, the immunoassay methods of the present invention as defined in the claims may preferably utilize a first antibody and/or a second antibody or antigen-binding fragment(s) or derivative(s) thereof being specific for (an) epitope(s) of histone H2A, wherein the first epitope and/or second epitope are epitopes of histone H2A present in the sequence spanning amino acid residues 20 to 118 of SEQ ID NO:2.

Further, the immunoassay methods of the present invention as defined in the claims may utilize a first antibody and/or a second antibody or antigen-binding fragment(s) or derivative(s) thereof being specific for (an) epitope(s) of histone H2A, wherein the first epitope and/or second epitope are epitopes of histone H2A present in the sequence spanning amino acid residues 21 to 53, 20 to 118 or 120 to 129 of SEQ ID NO:2.

Further, the immunoassay methods of the present invention as defined in the claims may utilize a first antibody and/or a second antibody or antigen-binding fragment(s) or derivative(s) thereof being specific for (an) epitope(s) of histone H3, wherein the first epitope and/or second epitope are epitopes of histone H3 present in the sequence spanning amino acid residues 27 to 62 of SEQ ID NO:3.

More preferably, the immunoassay methods of the present invention as defined in the claims may utilize a first antibody and/or a second antibody or antigen-binding fragment(s) or derivative(s) thereof being specific for (an) epitope(s) of histone H4, wherein the epitope(s) is/are selected from the group consisting of an amino acid sequence spanning residues 22 to 30 of SEQ ID NO:1, residues 46 to 56 of SEQ ID NO:1, residues 67 to 78 of SEQ ID NO:1, residues 92 to 102 of SEQ ID NO:1, residues 22 to 34 of SEQ ID NO: 1, and residues 46 to 102 of SEQ ID NO: 1.

Further, the immunoassay methods of the present invention as defined in the claims may utilize a first antibody and/or a second antibody or antigen-binding fragment(s) or derivative(s) thereof being specific for (an) epitope(s) of histone H2A, wherein the epitope(s) is/are selected from the group consisting of an amino acid sequence spanning residues 21 to 53 of SEQ ID NO:2, residues 21 to 29 of SEQ ID NO:2, residues 30 to 53 of SEQ ID NO:2, and residues 120 to 129 of SEQ ID NO: 2.

Further, the immunoassay methods of the present invention as defined in the claims may utilize a first antibody and/or a second antibody or antigen-binding fragment(s) or derivative(s) thereof being specific for (an) epitope(s) of histone H2B spanning residues 41 to 69 of SEQ ID NO: 4. Further, the immunoassay methods of the present invention may utilize a first antibody and/or a second antibody or antigen-binding fragment(s) or derivative(s) thereof being specific for (an) epitope(s) of histone H3 spanning residues 27 to 37 of SEQ ID NO: 3 and/or spanning residues 52 to 62 of SEQ ID NO: 3.

Further, the immunoassay methods of the present invention as defined in the claims may utilize a first antibody and/or the second antibody or the antigen-binding fragment or derivative thereof which are specific for an epitope of histone H2A present in the sequence spanning amino acid residues 21 to 53 and/or 120 to 129 of the histone H2A sequence represented by SEQ ID NO:2. Further, the immunoassay methods of the present invention as defined in the claims may utilize a first antibody, antigen-binding fragment or derivative thereof that is specific for an epitope of histone H4 present in the sequence spanning amino acid residues 22 to 102 of SEQ ID NO:1, and a second antibody, antigen-binding fragment or derivative thereof that is specific for an epitope of a free histone H2A, H2B, or preferably H3.

Further, the immunoassay methods of the present invention as defined in the claims may utilize a first antibody, antigen-binding fragment or derivative thereof that is specific for an epitope of histone H2B present in the sequence spanning amino acid residues 41 to 69 of SEQ ID NO:4, and a second antibody, antigen-binding fragment or derivative thereof that is specific for an epitope of a free histone H2A, H4, or H3.

Further, the immunoassay methods of the present invention as defined in the claims may utilize a first antibody, antigen-binding fragment or derivative thereof that is specific for an epitope of histone H2B present in the sequence spanning amino acid residues 20 to 118 of SEQ ID NO:2, and a second antibody, antigen-binding fragment or derivative thereof that is specific for an epitope of a free histone H2B, H4, or H3.

Further, the immunoassay methods of the present invention as defined in the claims may utilize a first antibody, antigen-binding fragment or derivative thereof that is specific for an epitope of histone H2B present in the sequence spanning amino acid residues 27 to 62 of SEQ ID NO:3, and a second antibody, antigen-binding fragment or derivative thereof that is specific for an epitope of a free histone H2B, H4, or H2A.

Further, the immunoassay methods of the present invention as defined in the claims may utilize a first antibody, antigen-binding fragment or derivative thereof that is specific for an epitope of histone H2A present in the sequence spanning amino acid residues 21 to 53, 120 to 129, or 20 to 118 of SEQ ID NO:2, and a second antibody, antigen-binding fragment or derivative thereof that is specific for an epitope of a free histone H3, H4 or preferably H2B.

Further disclosed herein is an antibody or an antigen-binding fragment or derivative thereof which is specific for an epitope of a histone protein or fragment thereof as detailed above. Exemplary antibodies that are successfully employed to detect histones or proADM, preferably MR-proADM are shown in the appended examples. The present invention as defined in the claims thus relates to an antibody(ies), (an) antigen-binding fragment(s) or derivative(s) thereof that is/are specific for an epitope of histone H2B, H4, H2A, H3 and/or proADM, preferably MR-proADM. Exemplary epitopes or peptides to which the antibodies are specifically binding to are herein documented above and below.

The level of the marker, e.g. the at least one histone and/or proADM, can also be determined by a mass spectrometric (MS) based analysis as described in the appended examples. Such a method may comprise detecting the presence, amount or concentration of one or more modified or unmodified fragment peptides of e.g. proADM and/or the histone in said biological sample or a protein digest (e.g. tryptic digest) from said sample, and optionally separating the sample with chromatographic methods, and subjecting the prepared and optionally separated sample to MS analysis. For example, selected reaction monitoring (SRM), multiple reaction monitoring (MRM) or parallel reaction monitoring (PRM) mass spectrometry may be used in the MS analysis, particularly to determine the amounts of at least one histone peptide. Herein, the term "mass spectrometry" or "MS" refers to an analytical technique to identify compounds by their mass. In order to enhance the mass resolving and mass determining capabilities of mass spectrometry, the samples can be processed prior to MS analysis. Accordingly, the invention as defined in the claims relates to MS detection methods that can be combined with immuno-enrichment technologies, methods related to sample preparation and/or chromatographic methods, preferably with liquid chromatography (LC), more preferably with high performance liquid chromatography (HPLC) or ultra high performance liquid chromatography (UHPLC). Sample preparation methods comprise techniques for lysis, fractionation, digestion of the sample into peptides, depletion, enrichment, dialysis, desalting, alkylation and/or peptide reduction. However, these steps are optional. The selective detection of analyte ions may be conducted with tandem mass spectrometry (MS/MS). Tandem mass spectrometry is characterized by mass selection step (as used herein, the term "mass selection" denotes isolation of ions having a specified m/z or narrow range of m/z's), followed by fragmentation of the selected ions and mass analysis of the resultant product (fragment) ions.

The skilled person is aware how quantify the level of a marker in the sample by mass spectrometric methods. For example, relative quantification "rSRM" or absolute quantification can be employed as described above.

"Prognosis" relates to the prediction of an outcome or a specific risk for a subject to experience/have/suffer from an adverse event, particularly mortality. This may also include an estimation of the chance of recovery or the chance of an adverse outcome for said subject.

Described herein but not claimed are also methods and kits for monitoring, therapy guidance and/or therapy control of subjects, the method comprises
(i) determining a level of at least one histone in a sample of said subject, and wherein said level of at least one histone is indicative of said adverse event of said subject; and/or
(ii) determining a level of proadrenomedullin (proADM) in a sample of said subject, and wherein said level of proADM is indicative of said adverse event of said subject.

The invention further relates to the use of kits and methods wherein such kits are used. The invention relates to the use of kits for carrying out the herein above and below provided methods. The herein provided definitions, e.g. provided in relation to the methods, also apply to the use of kits of the invention. In particular, the invention relates to use of kits for the prognosis, wherein said kit comprises
(i) detection reagents for determining said level of at least one histone in said sample of said subject, and
   reference data including said reference level of at least one histone, and wherein an increased level of said at least one histone in said sample of said subject as compared to said reference level of at least one histone is indicative of mortality of said subject occurring within 7 days; and
(ii) detection reagents for determining said level of proADM in said sample of said subject, and
   reference data including said reference level of proADM, and wherein an increased level of said proADM in said sample of said subject as compared to said reference level of proADM is indicative of mortality of said subject occurring within 7 days.

As used herein, "reference data" comprise reference level(s) of at least one histone and of proADM, particularly MR-proADM. The levels of the at least one histone and/or of proADM in the sample of the subject can be compared to the reference levels comprised in the reference data of the kit. An increased level of the marker(s) determined is indicative of the adverse event, particularly mortality. The reference levels are herein described above. The reference data can also include a reference sample to which the level of the at least one histone and/or the level of proADM is compared to. The reference data can also include an instruction manual how to use the kits of the invention.

As used herein, the "detection reagent" or the like are reagents that are suitable to determine the herein described marker(s), e.g. the at least one histone and/or the proADM. Such exemplary detection reagents are, for example, ligands, e.g. antibodies or fragments thereof, which specifically bind to the peptide or epitopes of the herein described marker(s). Such ligands might be used in immunoassays as described above. Further reagents that are employed in the immunoassays to determine the level of the marker(s) may also be comprised in the kit and are herein considered as detection reagents. Detection reagents can also relate to reagents that are employed to detect the markers or fragments thereof by MS based methods. Such detection reagent can thus also be reagents, e.g. enzymes, chemicals, buffers, etc, that are used to prepare the sample for the MS analysis. A mass spectrometer can also be considered as a detection reagent. Detection reagents according to the invention as defined in the claims can also be calibration solution(s), e.g. that can be employed to determine and compare the level of the marker(s).

As used herein, the terms "comprising" and "including" or grammatical variants thereof are to be taken as specifying at least the stated features, integers, steps or components but do not preclude the addition of one or more additional features, integers, steps, components or groups thereof. This term encompasses the terms "consisting of" and "consisting essentially of" that are understood to specify only the stated feature, integers, steps or components to the exclusion of any additional features.

Thus, the terms "comprising"/"including"/"having" mean that any further component (or likewise features, integers, steps and the like) can/may be present.

The term "consisting of" means that no further component (or likewise features, integers, steps and the like) is present.

The term "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Thus, the term "consisting essentially of" means those specific further components (or likewise features, integers, steps and the like) can be present, namely those not materially affecting the essential characteristics of the composition, device or method. In other words, the term "consisting essentially of" (which can be interchangeably used herein with the term "comprising substantially"), allows the presence of other components in the composition, device or method in addition to the mandatory components (or likewise features, integers, steps and the like), provided that the essential characteristics of the device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

The term "about" preferably refers to ±10% of the indicated numerical value, more preferably to ±5% of the indicated numerical value, and in particular to the exact numerical value indicated.

As used herein, the term "about" refers to ±10% of the indicated numerical value, and in particular to ±5% of the indicated numerical value. Whenever the term "about" is used, a specific reference to the exact numerical value indicated is also included. If the term "about" is used in connection with a parameter that is quantified in integers, such as the number of nucleotides in a given nucleic acid, the numbers corresponding to ±10% or ±5% of the indicated numerical value are to be rounded to the nearest integer. For example, the expression "about 25 amino acids" refers to the range of 23 to 28 amino acids, in particular the range of 24 to 26 amino acids, and preferably refers to the specific value of 25 amino acids.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy individuals not having a prenatal disorder or condition) and "disease" populations, e.g. subjects experiencing/having/suffering from an adverse event, e.g. mortality. For any particular marker (like MR-proADM), a distribution of marker levels for subjects with and without a disease/condition will likely overlap. Under such conditions, a test does not absolutely distinguish normal from disease with 100% accuracy, and the area of overlap might indicate where the test cannot distinguish normal from disease. A threshold is selected, below which the test is considered to be abnormal and above which the test is considered to be normal or below or above which the test indicates a specific condition, e.g. the abnormal event. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples (or adverse event) might be ranked according to degree (e.g. 1=low, 2=normal, and 3=high). This ranking can be correlated to results in the "normal" population, and a ROC curve created. These methods are well known in the art; see, e.g., Hanley et al. 1982. Radiology 143: 29-36. Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of the adverse event, particularly mortality. Thus, the area under the ROC curve can be used to determine the effectiveness of the test.

A positive likelihood ratio, negative likelihood ratio, odds ratio, or hazard ratio may be used as a measure of a test's ability to predict risk or diagnose a disorder or condition (adverse outcome), i.e. "diseased group". In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group.

The skilled artisan will understand that associating a diagnostic or prognostic indicator, with a diagnosis or with a prognostic risk of a future clinical outcome is a statistical analysis. For example, a marker level of lower than X may signal that a patient is more likely to suffer from an adverse event/outcome than patients with a level more than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value; see, e.g., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Unless otherwise indicated, established methods of recombinant gene technology were used as described, for example, in Sambrook, Russell "Molecular Cloning, A Laboratory Manual", Cold Spring Harbor Laboratory, N.Y. (2001).

### Example 1

### METHODS:

### Study population

Two hundred and thirty-seven critically ill patients admitted to the medical intensive care unit of 'centre hospitalier universitaire (CHU) de Dijon Bourgogne' from the 1^{st} of June 2013 to the 14^{th} of June 2014 were consecutively enrolled in the clinical study. Patients younger than 18 years were excluded. The study was approved by the local institutional review board. Before enrolment, written informed consent was obtained from patients themselves or from the patient's next of kin. All patients showed a broad spectrum of diseases including cardiovascular disease, diabetes mellitus, malignancy, respiratory disease, liver disease, renal disease and immunodepression and were monitored until discharge or death in the hospital. Based on retrospective review of medical records, imaging and microbiology results two independent physicians classified the patients on the day of admission as either non-sepsis (systemic inflammatory response syndrome (SIRS) or no SIRS), severe sepsis or septic shock according to international standardized criteria (Bone, Balk et al. 1992). A blood sample was taken on the day of admission, i.e. during the first 24 hours. Baseline demographics and clinical data including medical history, results from physical examination, routine blood analyses (e.g. blood cultures), non-laboratory diagnostic investigations (e.g. SIRS criteria, organ failure criteria), therapeutic interventions (e.g. mechanical ventilation (MV), vasopressors and renal replacement therapy (RRT)) as well as outcome parameters (e.g. length of stay, all cause mortality) were recorded. The sequential organ failure assessment (SOFA) score, based on six organ parameters, and the simplified acute physiology score (SAPS II), based on 17 mainly physiology variables, were calculated on admission (Le Gall, Lemeshow et al. 1993; Vincent, Moreno et al. 1996).

### Biomarkers

Serum lactate levels were measured by colorimetric assay using the e501 module analyser from Roche Diagnostics, Meylan, France. Reference limit for lactate was 0.5 - 2.2 mmol/L.

MR-proADM (midregional proadrenomedullin), copeptin and PCT (procalcitonin) levels were determined in plasma samples using ultrasensitive assays, such as KRYPTOR random access analyser (Thermo Scientific B·R·A·H·M·S). The levels of histone H2A, H2B, H3 and H4 as well as the level of Aldolase B were determined in the plasma samples by e.g. selected reaction monitoring or multiple reaction monitoring (SRM/MRM) assays as described in the following. Specific peptides derived from the markers were measured by LC-MS/MS technology (TSQ Quantiva mass spectrometer (MS); ThermoFisher Scientific). Identified peptide sequences and fragmentation ions thereof, so-called Transitions, for each peptide were found to be useful surrogates for monitoring marker proteins levels in a blood sample. Optimization was done on synthetic peptides which can be isotopically heavy labeled. Best peptides regarding signal to noise were selected. Optimal retention time and at least 4 best transitions were set up for each peptide.

### Exemplary MS Quantification and Choice of Peptides and Transitions

5uL of each clinical plasma sample was added to 20uL of 8M Urea/2.5% n-propanol/300mM Tris/10mM DTT pH 8.5 and incubated at 37C for one hour. 500mM iodoacetic acid prepared in 1M ammonium bicarbonate was added to each sample well and incubated in the dark at room temperature for one hour. 113uL of 50mM Tris/5mM CaCl2 pH 8.0 were added to each well. Trypsin (Thermo Fisher Scientific) was rehydrated with 150uL of 25mM acetic acid is added with a ratio 1:10 (total protein content:protease) and incubated at 37C for 20 hours. Digestion was finally quenched with the additional of 2uL of formic acid. Glucagon (1ng/uL) and standard heavy peptides were then added before injection.

SRM assays were developed on a triple quadrupole mass spectrometer TSQ Quantiva coupled with HPLC Ultimate 3000 (Thermo Fisher Scientific). Reverse phase separations were carried out in a 20 min linear gradient from 5 to 40% B, with a total run time of 40 min (Solvent A : Water 0.2% FA, Solvent B : ACN 0.2% FA). The flow rate during the linear gradient was set to 240 *µ*L/min. The total injection volume was 160 *µ*L for all samples and points on the curve. A 150 mm ×2.1 mm Accucore aQ column (ThermoFisher Scientific) was run at a temperature of 50°C.

Optimization was performed on heavy labeled synthetic peptides, incorporating 13C- and 15N-labeled arginine or lysine (ThermoFisher Scientific or New England Peptide). Individual instrument parameters such as collision energy, tube lens, and dwell time were automatically tested for every transition. After multiple iterations, the optimized list of peptides and transitions (i.e. highest intensity signal and least overlap with other transitions), and corresponding retention times were finalized with at least four fragment transitions per peptide chosen.

Peptides were identified by co-eluting light and heavy-labeled transitions in the chromatographic separation. Pinpoint (Thermo Fisher Scientific) and Skyline (MacCoss Lab) softwares were used for time alignment, relative quantification of the transitions and targeted protein quantification.

Relative and absolute quantifications of the markers were performed by employing the exemplary methods as described in the following.

### Relative quantification:

1. Determining increased or decreased presence of the marker by comparing the SRM signature peak area from a given peptide detected in biological sample to the same SRM signature peak area of the same fragment peptide in at least a second, third, fourth or more biological samples.
2. Determining increased or decreased presence of the marker by comparing the SRM signature peak area from a given peptide detected in a biological sample to SRM signature peak areas developed from fragment peptides from other proteins, in other samples derived from different and separate biological sources, where the SRM signature peak area comparison between the 2 samples for a peptide fragment are normalized to amount of protein analyzed in each sample.
3. Determining increased or decreased presence of the marker by comparing the SRM signature peak area for a given peptide to the SRM signature peak areas from other fragment peptides derived from different proteins within the same biological sample in order to normalize changing levels of the maker to levels of other proteins that do not change their levels of expression under various cellular conditions.
4. These assays were applied to both unmodified fragment peptides and for modified fragment peptides, e.g. the histones protein, where the modifications included, but were not limited to phosphorylation and/or glycosylation, acetylation, methylation (mono, di, tri), citrullination, ubiquitinization and where the relative levels of modified peptides were determined in the same manner as determining relative amounts of unmodified peptides.

### Absolute quantification of a given peptide:

Comparing the SRM/MRM signature peak area for a given fragment peptide from the marker in an individual biological sample to the SRM/MRM signature peak area of an internal fragment peptide standard spiked into the protein lysate from the biological sample.

The internal standard was a labeled synthetic version of the fragment peptide from the marker protein that was being interrogated or the labeled recombinant protein. This standard was spiked into a sample in known amounts before or after digestion, and the SRM/MRM signature peak area was determined for both the internal fragment peptide standard and the native fragment peptide in the biological sample separately, followed by comparison of both peak areas.

Such an assay was applied to unmodified fragment peptides and modified fragment peptides, where the modifications included but are not limited to phosphorylation and/or glycosylation, acetylation, methylation (mono, di, tri), citrullination, ubiquitinization, and where the absolute levels of modified peptides were determined in the same manner as determining absolute levels of unmodified peptides.

Histone H4 was also measured by an immunoassay. The Histone H4 Immuno-Assay (H4 IA) consist of a mouse monoclonal antibody (mAb) raised against a synthetic peptide (amino acids 46-56 of SEQ ID NO: 1) coupled to MagPlex-C Micropheres (Luminex, Austin Texas), and a biotinylated sheep polyclonal antibody (pAb) raised against a synthetic peptide (amino acids 67-78 of SEQ ID NO 1). A synthetic peptide (amino acids 46-102 of SEQ ID NO: 1) was used as standard material. Samples were measured on a MAgPix with xPonent 4.2 System (Luminex, Austin Texas). Data was analyzed using 5 parameter logistic regression from JMP-12 (SAS statistical software, UK).

### Statistical analysis

All analyses were performed using the software R 3.0.2.

The data is expressed as median and interquartile range [IQR] in brackets.

As all analyzed biomarkers show highly right-skewed distributions, values were log₁₀-transformed prior to inclusion into regression models in order to decrease the impact of extreme values on the model fit.

Values below limit of quantification (LoQ) were replaced by a small value below LoQ. Missing values were not replaced. Each model includes all patients with complete data on all variables in the model.

P-values <0.05 were considered as significant.

For survival analyses, follow-up was censored at 3, 7 or 28 days after ICU admission (maximal follow-up period (FUP)), as appropriate. Patients lost to follow-up before the evaluated FUP (i.e. due to early discharge or relocation to a different ward) were censored at the day of their last visit on the ICU. Patients alive at the maximal FUP were censored at this day. For time-dependent outcome variables, Cox regression models were used. Displayed results are the Likelihood-Ratio-χ² test (L.R. χ² and p-value), C index (Harrel) and standardized hazard ratios (HR). Hazard ratios herein refer to a two-fold change in the biomarkers level (upper vs. lower quartile of biomarkers). In adjusted models, adjusting variables were included into the model in order to determine the additional effect of biomarkers on model performance.

### Results:

The study population comprised 237 patients. Two patients (one patient without SIRS, one sepsis patient) had to be excluded from analyses due to conflicting documentation of mortality data. One hundred and seventy-two patients (73 %) presented with severe sepsis or septic shock, 15 patients (6 %) with SIRS and 49 patients (21 %) without SIRS. Median age was 67 [59-77 years] years. The majority of patients was male (60 %). Most frequent underlying conditions were cardiovascular diseases (35 %), diabetes mellitus (31 %) and malignancies (27 %) followed by respiratory disease (16 %), liver disease (12 %), renal disease (12 %) and immunodepression (7 %). Most frequent site of infection was the lower respiratory tract (46 %) and urinary tract (45 %). SAPS II score (56 [40-69 points] points) and SOFA score (9 [6-12 points] points) were increased on admission. Organ failures were most frequently respiratory failure (61 %), circulatory shock (56 %) and renal failure (41 %). Accordingly, many patients required MV (78 %), vasopressors (68 %) and RRT (37 %) during ICU stay. All cause ICU mortality was 32 %, median length of ICU stay was 5.4 [2.5-10.6] days.

We analyzed short term (e.g. at day 3 and 7, i.e. 3 day and 7 day) and long term (at day 28, i.e. 28 day) mortality in all 235 critically ill patients using uni- and bivariate Cox regression models adjusted for age and sex. Twenty-three patients (10 %) had died by day 3, 49 patients (21 %) had died by day 7 and 74 patients (32 %) had died by day 28 after ICU admission. In addition, mortality was analyzed in subpopulations of patients with lower respiratory tract infection, urinary tract infection (UTI) and malignancies using univariate Cox regression models adjusted for age and sex. Twenty-seven patients (25 %) of 109 patients with lower respiratory tract infection had died by day 7, 34 patients (36 %) of 94 patients with UTI had died by day 28 and 16 patients (25 %) of 64 patients with malignancies had died by day 7 after ICU admission. The power of each Cox regression model to discriminate survivors from non-survivors is reflected by the C index ranging from 0 to 1 with best predictive Cox regression models resulting in a C index close to 1. In addition, HR are calculated with HR>0 indicating a worse prognosis and HR<0 indicating a protective effect of the variable.

Among all analyzed variables, the SAPS II score on ICU admission shows the best prediction at 3 day (C index 0.876, HR per IQR 8.42), 7 day (C index 0.809, HR per IQR 5.15) and 28 day (C index 0.776, HR per IQR 4.19) mortality, followed by the SOFA score on ICU admission in prediction of 3 day (C index 0.866, HR per IQR 6.68) and 7 day mortality (C index 0.778, HR per IQR 3.82) in all 235 critically ill patients (Table 1 - Table 3). Similar results are obtained in the subgroup of sepsis patients and for prediction of 7 day mortality in critically ill patients with lower respiratory tract infection for the SAPS II score (C index 0.773, HR per IQR 3.47) (Table 4).

In comparison, MR-proADM discriminates survivors and non-survivors best among all biomarkers on day 7 (C index 0.769, HR per IQR 4.53) and day 28 (C index 0.765, HR per IQR 4.86) after ICU admission in all critically ill patients (Table 2, Table 3). It is superior to the SOFA score in predicting 28d mortality in all critically ill patients (Table 3). Similar results are obtained in the subgroup of sepsis patients. In bivariate analysis, MR-proADM improves the performance of SAPS II or SOFA for prediction of 7 day mortality (C index 0.832 for combined model of SAPS II and MR-proADM compared to C index 0.809 for SAPS II alone) and the performance of SAPS II for prediction of 28 day mortality (C index 0.810 for combined model of SAPS II and MR-proADM compared to C index 0.776 for SAPS II alone) in all critically ill patients (Table 2, Table 3). There is no improved prognostic value in a combined model of SAPS II or SOFA and MR-proADM for prediction of 3 day mortality in these patients (Table 1).

In comparison to other biomarkers and scores, PCT (e.g C index 0.689, HR per IQR 1.90 for prediction of 28 day mortality in all critically ill patients) and aldolase B (e.g. C index 0.667, HR per IQR 1.47 for prediction of 28 day mortality in all critically ill patients) on ICU admission show moderate association with mortality in all critically ill patients or subgroups thereof (e.g. Table 1). However, in a bivariate Cox regression model PCT improves the prognostic value of SAPS II or SOFA for prediction of 28 day mortality in all critically ill patients (e. g. combined model of SAPS II and PCT results in a C index of 0.786 compared to 0.776 for SAPS II in univariate analysis) (e.g. Table 3). In addition, a bivariate Cox regression model including aldolase B improves association of MR-proADM with 7 day mortality in all critically ill patients (combined model of MR-proADM and aldolase B with a C index of 0.780 compared to univariate model of MR-proADM with a C index of 0.769) (Table 2). The prediction was not further improved by PCT or aldolase B to SAPS II, MR-proADM or histones in other mortality analyses (Table 1 - Table 3).

The levels of the histones H2A, H2B, H3 and H4 on ICU admission were strongly associated with 3 day (e.g. H2B C index 0.793, HR per IQR 2.76), 7 day (e.g. H2B C index 0.768, HR per IQR 2.40) and 28 day (e.g. H2B C index 0.752, HR per IQR 2.40) mortality in all critically ill patients (Table 1 - Table 3). Similar results are obtained in the subgroup of sepsis patients. Among all histones H2B performs best, followed by H4, H2A and H3 (Table 1 - Table 6). Comparing the performance of histones H2A, H2B, H3 and H4 to other biomarkers, there is a striking prognostic value of histones for short term (3 and 7 day) mortality, i.e. while H2B may be inferior to MR-proADM in prediction of 28 day mortality (MR-proADM C index 0.765 versus H2B C index 0.752). H2B and MR-proADM are comparably associated with 7 day mortality (H2B C index 0.793 versus MR-proADM C index 0.786) and H2B is superior to MR-proADM for prediction of 3 day mortality (H2B C index 0.768 versus MR-proADM C index 0.769) in all critically ill patients (Table 1 - Table 3).

In bivariate Cox regression models, histones H2A, H2B, H3 and H4 improve the performance of SAPS II or SOFA (e.g. combined model of H2B and SAPS II C index 0.811 compared to univariate model of SAPS II C index 0.776 for prediction of 28 day mortality) and MR-proADM (combined model of H2B and MR-proADM C index 0.795 compared to univariate model of MR-proADM C index 0.765 for prediction of 28 day mortality) (Table 1 - Table 3). In critically ill patients with lower respiratory tract infection histones H2A, H2B, H3 and H4 on ICU admission are the best predictor of 7 day mortality among the biomarkers (e.g. H2B C index 0.785, HR per IQR 2.56) (Table 4). In critically ill patients with UTI histones H2A, H2B, H3 and H4 are strongest associated with 28 day mortality among all variables on ICU admission (e.g. H2B C index 0.764, HR per IQR 2.52) (Table 5). Histones H2A, H2B, H3 and H4 on ICU admission show strongest association with 7 day mortality (e.g. H2B C index 0.815, HR per IQR 4.79) among all variables in critically ill patients admitted to the ICU with malignancies (Table 6).

### Table 1: Uni- and bivariable Cox regression analysis for 3 day mortality in all critically patients

Twenty-four patients of a total number (n) of 235 critically ill patients had died by day 3 after ICU admission (events). Scores or biomarkers measured on ICU admission are included in the models. All univariable or bivariable models are adjusted for age and sex. The degrees of freedom (df) reflect the number of variables and adjustments included. Displayed results are the Likelihood-Ratio-χ² test (L.R. χ² and p-value), C index (Harrel) and standardized hazard ratios (HR) plus 95 % confidence interval (CI), either per interquartile range (IQR) or 2fold change. (SAPS II: simplified acute physiology score II; SOFA: sequential organ failure assessment; MR-proADM: midregional proadrenomedullin; PCT: procalcitonin; Histones are represented by histone H2A, H2B, H3 and H4; H4 was also measured by an immunoassay (IA)).

**Table 1**

| **Model** | **N** | **Event s** | **L.R. χ²** | **d f** | **p-value** | **C index** | **HR [95 % Cl] per ...** | |
|---|---|---|---|---|---|---|---|---|
| | | | | | | | **... IQR** | **... 2fold change** |
| **SAPS II** | 235 | 24 | 55.11 | 3 | <0.001 | 0.876 | 8.42 [4.38-16.19] | |
| **SOFA** | 235 | 24 | 41.78 | 3 | <0.001 | 0.866 | 6.68 [3.33-13.39] | |
| **H2B** | 235 | 24 | 29.31 | 3 | <0.001 | 0.793 | 2.76 [1.77-4.29] | 1.47 [1.24-1.73] |
| **H4** | 235 | 24 | 28.24 | 3 | <0.001 | 0.786 | 2.70 [1.72-4.25] | 1.45 [1.23-1.72] |
| **H2A** | 235 | 24 | 28.14 | 3 | <0.001 | 0.787 | 2.45 [1.63-3.66] | 1.45 [1.23-1.72] |
| **H4 IA** | 228 | 23 | 26.61 | 3 | <0.001 | 0.779 | 14.35 [3.26-63.19] | 1.39 [1.16-1.66] |
| **MR-proADM** | 235 | 24 | 24.99 | 3 | <0.001 | 0.786 | 4.82 [2.08-11.18] | 2.05 [1.40-3.01] |
| **H3** | 235 | 24 | 19.68 | 3 | <0.001 | 0.740 | 3.45 [1.54-7.70] | 1.23 [1.08-1.41] |
| **Aldolase B** | 235 | 24 | 14.65 | 3 | 0.002 | 0.721 | 1.65 [1.05-2.59] | 1.16 [1.02-1.32] |
| **PCT** | 235 | 24 | 12.08 | 3 | 0.007 | 0.705 | 1.49 [0.85-2.62] | 1.09 [0.97-1.23] |
| **H2B + SAPS** II | 235 | 24 | 61.95 | 4 | <0.001 | 0.885 | | |
| **H4 + SAPS II** | 235 | 24 | 61.83 | 4 | <0.001 | 0.884 | | |
| **H2A + SAPS** II | 235 | 24 | 61.70 | 4 | <0.001 | 0.884 | | |
| **H4 IA + SAPS** II | 228 | 23 | 61.51 | 4 | <0.001 | 0.898 | | |
| **H3 + SAPS II** | 235 | 24 | 57.02 | 4 | <0.001 | 0.878 | | |
| **MR-proADM** + **SAPS II** | 235 | 24 | 56.79 | 4 | <0.001 | 0.884 | | |
| **PCT + SAPS** II | 234 | 24 | 55.10 | 4 | <0.001 | 0.875 | | |
| **Aldolase B + SAPS II** | 234 | 24 | 54.97 | 4 | <0.001 | 0.876 | | |
| **MR-proADM** + **H2B** | 235 | 24 | 36.15 | 4 | <0.001 | 0.828 | | |
| **H4 IA + SOFA** | 228 | 23 | 50.05 | 4 | <0.001 | 0.887 | | |
| **H2B + SOFA** | 235 | 24 | 47.98 | 4 | <0.001 | 0.876 | | |
| **H4 + SOFA** | 235 | 24 | 47.56 | 4 | <0.001 | 0.876 | | |
| **H2A + SOFA** | 235 | 24 | 47.30 | 4 | <0.001 | 0.874 | | |
| **H3 + SOFA** | 235 | 24 | 43.63 | 4 | <0.001 | 0.863 | | |
| **MR-proADM+ SOFA** | 235 | 24 | 42.27 | 4 | <0.001 | 0.872 | | |
| **PCT + SOFA** | 234 | 24 | 42.04 | 4 | <0.001 | 0.866 | | |
| **Aldolase B + SOFA** | 234 | 24 | 41.60 | 4 | <0.001 | 0.865 | | |
| **MR-proADM + H4** | 235 | 24 | 35.29 | 4 | <0.001 | 0.826 | | |
| **MR-proADM + H2A** | 235 | 24 | 35.16 | 4 | <0.001 | 0.825 | | |
| **MR-proADM + H4 IA** | 228 | 23 | 32.60 | 4 | <0.001 | 0.809 | | |
| **MR-proADM + H3** | 235 | 24 | 29.15 | 4 | <0.001 | 0.806 | | |
| **H2B + Aldolase B** | 234 | 24 | 31.35 | 4 | <0.001 | 0.799 | | |
| **PCT + H2B** | 234 | 24 | 29.29 | 4 | <0.001 | 0.791 | | |

### Table 2: Uni- and bivariable Cox regression analysis for 7 day mortality in all critically patients

Fourty-nine patients of a total number (n) of 235 critically ill patients had died by day 7 after ICU admission (events). Scores or biomarkers measured on ICU admission are included in the models. All univariable or bivariable models are adjusted for age and sex. The degrees of freedom (df) reflect the number of variables and adjustments included. Displayed results are the Likelihood-Ratio-χ² test (L.R. χ² and p-value), C index (Harrel) and standardized hazard ratios (HR) plus 95 % confidence interval (CI), either per interquartile range (IQR) or 2fold change. (SAPS II: simplified acute physiology score II; SOFA: sequential organ failure assessment MR-proADM: midregional proadrenomedullin; PCT: procalcitonin; Histones are represented by histone H2A, H2B, H3 and H4; H4 was also measured by an immunoassay (IA)).

**Table 2**

| **Model** | **N** | **Event s** | **L.R. χ²** | **d f** | **p-value** | **C index** | **HR [95 % Cl] per ...** | |
|---|---|---|---|---|---|---|---|---|
| | | | | | | | **... IQR** | **... 2fold change** |
| **SAPS II** | 235 | 49 | 68.07 | 3 | <0.001 | 0.809 | 5.15 [3.26-8.13] | |
| **SOFA** | 235 | 49 | 50.64 | 3 | <0.001 | 0.778 | 3.82 [2.39-6.09] | |
| **MR-proADM** | 235 | 49 | 45.88 | 3 | <0.001 | 0.769 | 4.53 [2.55-8.04] | 1.99 [1.53-2.59] |
| **H2B** | 235 | 49 | 45.33 | 3 | <0.001 | 0.768 | 2.40 [1.76-3.28] | 1.39 [1.24-1.57] |
| **H4** | 235 | 49 | 42.81 | 3 | <0.001 | 0.761 | 2.32 [1.68-3.18] | 1.37 [1.22-1.55] |
| **H2A** | 235 | 49 | 40.91 | 3 | <0.001 | 0.755 | 2.09 [1.57-2.78] | 1.36 [1.21-1.54] |
| **H3** | 235 | 49 | 38.22 | 3 | <0.001 | 0.742 | 3.62 [2.07-6.34] | 1.24 [1.13-1.37] |
| **H4 IA** | 228 | 48 | 35.98 | 3 | <0.001 | 0.741 | 5.57 [2.40-12.96] | 1.23 [1.11-1.37] |
| **Aldolase B** | 235 | 49 | 26.66 | 3 | <0.001 | 0.697 | 1.69 [1.22-2.33] | 1.17 [1.06-1.28] |
| **PCT** | 235 | 49 | 23.57 | 3 | <0.001 | 0.698 | 1.66 [1.12-2.46] | 1.12 [1.02-1.22] |
| **H2B + SAPS II** | 235 | 49 | 83.39 | 4 | <0.001 | 0.839 | | |
| **H4 + SAPS II** | 235 | 49 | 82.29 | 4 | <0.001 | 0.834 | | |
| **H2A + SAPS II** | 235 | 49 | 80.74 | 4 | <0.001 | 0.830 | | |
| **H3 + SAPS II** | 235 | 49 | 79.27 | 4 | <0.001 | 0.835 | | |
| **MR-proADM + SAPS II** | 235 | 49 | 76.75 | 4 | <0.001 | 0.832 | | |
| **H4 IA + SAPS II** | 228 | 48 | 75.64 | 4 | <0.001 | 0.834 | | |
| **Aldolase B + SAPS II** | 234 | 49 | 70.32 | 4 | <0.001 | 0.809 | | |
| **PCT + SAPS II** | 234 | 49 | 68.47 | 4 | <0.001 | 0.811 | | |
| **H2B + SOFA** | 235 | 49 | 61.45 | 4 | <0.001 | 0.810 | | |
| **H4 + SOFA** | 235 | 49 | 60.08 | 4 | <0.001 | 0.804 | | |
| **H4 IA + SOFA** | 228 | 48 | 59.58 | 4 | <0.001 | 0.803 | | |
| **MR-proADM + H2B** | 235 | 49 | 59.50 | 4 | <0.001 | 0.804 | | |
| **H2A + SOFA** | 235 | 49 | 58.66 | 4 | <0.001 | 0.800 | | |
| **H3 + SOFA** | 235 | 49 | 58.44 | 4 | <0.001 | 0.800 | | |
| **MR-proADM + H4** | 235 | 49 | 57.83 | 4 | <0.001 | 0.801 | | |
| **MR-proADM + SOFA** | 235 | 49 | 57.26 | 4 | <0.001 | 0.800 | | |
| **MR-proADM + H2A** | 235 | 49 | 56.25 | 4 | <0.001 | 0.796 | | |
| **MR-proADM + H3** | 235 | 49 | 55.10 | 4 | <0.001 | 0.795 | | |
| **MR-proADM + H4 IA** | 228 | 48 | 52.66 | 4 | <0.001 | 0.780 | | |
| **Aldolase B + SOFA** | 234 | 49 | 51.39 | 4 | <0.001 | 0.781 | | |
| **PCT + SOFA** | 234 | 49 | 50.77 | 4 | <0.001 | 0.778 | | |
| **MR-proADM+ Aldolase B** | 234 | 49 | 49.84 | 4 | <0.001 | 0.780 | | |
| **PCT + H2B** | 234 | 49 | 46.55 | 4 | <0.001 | 0.769 | | |
| **MR-proADM + PCT** | 234 | 49 | 46.37 | 4 | <0.001 | 0.768 | | |
| **H2B + Aldolase B** | 234 | 49 | 46.13 | 4 | <0.001 | 0.765 | | |

### Table 3: Uni- and bivariable Cox regression analysis for 28 day mortality in all critically ill patients

Seventy-four patients of a total number (n) of 235 critically ill patients had died by day 3 after ICU admission (events). Scores or biomarkers measured on ICU admission are included in the models. All univariable or bivariable models are adjusted for age and sex. The degrees of freedom (df) reflect the number of variables and adjustments included. Displayed results are the Likelihood-Ratio-χ² test (L.R. χ² and p-value), C index (Harrel) and standardized hazard ratios (HR) plus 95 % confidence interval (CI), either per interquartile range (IQR) or 2fold change. (SAPS II: simplified acute physiology score II; SOFA: sequential organ failure assessment MR-proADM: midregional proadrenomedullin; PCT: procalcitonin; Histones are represented by histone H2A, H2B, H3 and H4; H4 was also measured by an immunoassay (IA))

**Table 3**

| **Model** | **N** | **Event s** | **L.R. χ²** | **d f** | **p-value** | **C inde x** | **HR [95 % Cl] per ...** | |
|---|---|---|---|---|---|---|---|---|
| | | | | | | | **... IQR** | **... 2fold change** |
| **SAPS II** | 235 | 74 | 76.19 | 3 | <0.00 1 | 0.776 | 4.19 [2.89-6.07] | |
| **MR-proADM** | 235 | 74 | 66.81 | 3 | <0.00 1 | 0.765 | 4.86 [3.05-7.73] | 2.06 [1.66-2.54] |
| **SOFA** | 235 | 74 | 59.04 | 3 | <0.00 1 | 0.753 | 3.33 [2.27-4.881 | |
| **H2B** | 235 | 74 | 58.04 | 3 | <0.00 1 | 0.752 | 2.40 [1.84-3.11] | 1.39 [1.26-1.54] |
| **H4** | 235 | 74 | 53.89 | 3 | <0.00 1 | 0.742 | 2.28 [1.75-2.99] | 1.37 [1.23-1.51] |
| **H2A** | 235 | 74 | 53.09 | 3 | <0.00 1 | 0.742 | 2.11 [1.66-2.70] | 1.37 [1.24-1.52] |
| **H4 IA** | 228 | 70 | 48.71 | 3 | <0.00 1 | 0.734 | 6.04 [2.97-12.27] | 1.25 [1.14-1.36] |
| **H3** | 235 | 74 | 45.95 | 3 | <0.00 1 | 0.720 | 3.33 [2.10-5.29] | 1.22 [1.13-1.32] |
| **PCT** | 235 | 74 | 33.87 | 3 | <0.00 1 | 0.689 | 1.90 [1.37-2.64] | 1.15 [1.07-1.24] |
| **Aldolase B** | 235 | 74 | 25.91 | 3 | <0.00 1 | 0.667 | 1.47 [1.11-1.95] | 1.12 [1.03-1.22] |
| **H2B + SAPS II** | 235 | 74 | 100.7 5 | 4 | <0.00 1 | 0.811 | | |
| **H4 + SAPS II** | 235 | 74 | 98.37 | 4 | <0.00 1 | 0.806 | | |
| **H2A + SAPS II** | 235 | 74 | 97.64 | 4 | <0.00 1 | 0.805 | | |
| **MR-proADM + SAPS II** | 235 | 74 | 97.47 | 4 | <0.00 1 | 0.810 | | |
| **H3 + SAPS II** | 235 | 74 | 93.05 | 4 | <0.00 1 | 0.801 | | |
| **H4 IA + SAPS II** | 228 | 70 | 90.73 | 4 | <0.00 1 | 0.804 | | |
| **MR-proADM + H2B** | 235 | 74 | 83.70 | 4 | <0.00 1 | 0.795 | | |
| **PCT + SAPS II** | 234 | 74 | 81.63 | 4 | <0.00 1 | 0.786 | | |
| **MR-proADM + H4** | 235 | 74 | 81.25 | 4 | <0.00 1 | 0.792 | | |
| **MRproADM + H2A** | 235 | 74 | 80.39 | 4 | <0.00 1 | 0.789 | | |
| **H2B + SOFA** | 235 | 74 | 77.41 | 4 | <0.00 1 | 0.785 | | |
| **MR-proADM + SOFA** | 235 | 74 | 77.32 | 4 | <0.00 1 | 0.783 | | |
| **Aldolase B + SAPS** II | 234 | 74 | 77.24 | 4 | <0.00 1 | 0.774 | | |
| **MR-proADM + H3** | 235 | 74 | 76.68 | 4 | <0.00 1 | 0.786 | | |
| **MR-proADM + H4** IA | 228 | 70 | 76.13 | 4 | <0.00 1 | 0.784 | | |
| **H4 + SOFA** | 235 | 74 | 74.89 | 4 | <0.00 1 | 0.778 | | |
| **H2A + SOFA** | 235 | 74 | 74.08 | 4 | <0.00 1 | 0.776 | | |
| **H4 IA + SOFA** | 228 | 70 | 72.78 | 4 | <0.00 1 | 0.778 | | |
| **H3 + SOFA** | 235 | 74 | 71.09 | 4 | <0.00 1 | 0.770 | | |
| **MR-proADM** + **Aldolase B** | 234 | 74 | 68.19 | 4 | <0.00 1 | 0.771 | | |
| **MR-proADM + PCT** | 234 | 74 | 66.57 | 4 | <0.00 1 | 0.766 | | |
| **PCT + SOFA** | 234 | 74 | 62.80 | 4 | <0.00 1 | 0.756 | | |
| **PCT + H2B** | 234 | 74 | 62.73 | 4 | <0.00 1 | 0.757 | | |
| **H2B + Aldolase B** | 234 | 74 | 62.54 | 4 | <0.00 1 | 0.753 | | |
| **Aldolase B + SOFA** | 234 | 74 | 59.13 | 4 | <0.00 1 | 0.753 | | |

### Table 4: Univariable Cox regression analysis for 7 day mortality in critically ill patients with lower respiratory tract infection

Twenty-seven patients of a total number (n) of 109 critically ill patients with lower respiratory tract infection had died by day 7 after ICU admission (events). Scores or biomarkers measured on ICU admission are included in the models. All univariable or bivariable models are adjusted for age and sex. The degrees of freedom (df) reflect the number of variables and adjustments included. Displayed results are the Likelihood-Ratio-χ² test (L.R. χ² and p-value), C index (Harrel) and standardized hazard ratios (HR) plus 95 % confidence interval (CI), either per interquartile range (IQR) or 2fold change. (SAPS II: simplified acute physiology score II; SOFA: sequential organ failure assessment MR-proADM: midregional proadrenomedullin; PCT: procalcitonin; Histones are represented by histone H2A, H2B, H3 and H4 was also measured by an immunoassay (IA))

**Table 4**

| **Model** | **N** | **Events** | **L.R. χ²** | **df** | **p-value** | **C index** | **HR [95 % Cl] per ...** | |
|---|---|---|---|---|---|---|---|---|
| | | | | | | | **... IQR** | **... 2fold change** |
| **SAPS II** | 109 | 27 | 29.14 | 3 | <0.001 | 0.773 | 3.47 [1.86-6.49] | |
| **H2B** | 109 | 27 | 27.70 | 3 | <0.001 | 0.785 | 2.56 [1.59-4.12] | 1.39 [1.18-1.64] |
| **H4** | 109 | 27 | 26.36 | 3 | <0.001 | 0.778 | 2.39 [1.49-3.84] | 1.36 [1.15-1.61] |
| **H2A** | 109 | 27 | 25.40 | 3 | <0.001 | 0.774 | 2.16 [1.40-3.33] | 1.35 [1.14-1.60] |
| **H4 IA** | 105 | 26 | 22.06 | 3 | <0.001 | 0.752 | 5.64 [1.66-19.13] | 1.23 [1.06-1.41] |
| **SOFA** | 109 | 27 | 21.84 | 3 | <0.001 | 0.742 | 2.72 [1.38-5.36] | 1.05 [1.02-1.09] |
| **MR-proADM** | 109 | 27 | 21.54 | 3 | <0.001 | 0.752 | 2.86 [1.37-5.94] | 1.67 [1.17-2.39] |
| **H3** | 109 | 27 | 21.48 | 3 | <0.001 | 0.760 | 2.89 [1.36-6.13] | 1.19 [1.05-1.35] |
| **Aldolase B** | 109 | 27 | 17.16 | 3 | 0.001 | 0.721 | 1.49 [1.00-2.22] | 1.14 [1.00-1.29] |
| **PCT** | 109 | 27 | 14.81 | 3 | 0.002 | 0.716 | 1.47 [0.77-2.80] | 1.08 [0.95-1.22] |

### Table 5: Univariable Cox regression analysis for 28 day mortality in critically ill patients with urinary tract infection

Thirty-four patients of a total number (n) of 94 critically ill patients with urinary tract infection had died by day 28 after ICU admission (events). Scores or biomarkers measured on ICU admission are included in the models. All univariable or bivariable models are adjusted for age and sex. The degrees of freedom (df) reflect the number of variables and adjustments included. Displayed results are the Likelihood-Ratio-χ² test (L.R. χ² and p-value), C index (Harrel) and standardized hazard ratios (HR) plus 95 % confidence interval (CI), either per interquartile range (IQR) or 2fold change. (SAPS II: simplified acute physiology score II; SOFA: sequential organ failure assessment MR-proADM: midregional proadrenomedullin; PCT: procalcitonin; Histones are represented by histone H2A, H2B, H3 and H4; H4 was also measured by an immunoassay (IA))

**Table 5**

| **Model** | **N** | **Events** | **L.R. χ²** | **df** | **p-value** | **C index** | **HR [95 % Cl] per ...** | |
|---|---|---|---|---|---|---|---|---|
| | | | | | | | **... IQR** | **... 2fold change** |
| **H2B** | 94 | 34 | 25.79 | 3 | <0.001 | 0.764 | 2.52 [1.75-3.65] | 1.39 [1.22-1.59] |
| **H4** | 94 | 34 | 25.46 | 3 | <0.001 | 0.761 | 2.47 [1.70-3.60] | 1.38 [1.21-1.58] |
| **H3** | 94 | 34 | 24.98 | 3 | <0.001 | 0.756 | 4.52 [2.34-8.75] | 1.29 [1.15-1.44] |
| **H2A** | 94 | 34 | 24.65 | 3 | <0.001 | 0.759 | 2.32 [1.64-3.28] | 1.39 [1.21-1.59] |
| **SAPS II** | 94 | 34 | 23.69 | 3 | <0.001 | 0.737 | 3.00 [1.85-4.89] | |
| **H4 IA** | 90 | 31 | 22.52 | 3 | <0.001 | 0.751 | 8.43 [2.79-25.45] | 1.29 [1.13-1.47] |
| **SOFA** | 94 | 34 | 16.07 | 3 | 0.001 | 0.696 | 2.28 [1.39-3.76] | 1.05 [1.02-1.08] |
| **MR-proADM** | 94 | 34 | 14.93 | 3 | 0.002 | 0.689 | 2.28 [1.36-3.81] | 1.65 [1.21-2.25] |
| **Aldolase B** | 94 | 34 | 11.96 | 3 | 0.008 | 0.679 | 1.82 [1.19-2.78] | 1.18 [1.05-1.33] |
| **PCT** | 94 | 34 | 5.54 | 3 | 0.136 | 0.594 | 1.30 [0.74-2.29] | 1.05 [0.94-1.17] |

### Table 6: Univariable Cox regression analysis for 7 day mortality in critically ill patients with malignancies

Sixteen patients of a total number (n) of 64 critically ill patients with malignancies had died by day 7 after ICU admission (events). Scores or biomarkers measured on ICU admission are included in the models. All univariable or bivariable models are adjusted for age and sex. The degrees of freedom (df) reflect the number of variables and adjustments included. Displayed results are the Likelihood-Ratio-χ² test (L.R. χ² and p-value), C index (Harrel) and standardized hazard ratios (HR) plus 95 % confidence interval (CI), either per interquartile range (IQR) or 2fold change. (SAPS II: simplified acute physiology score II; SOFA: sequential organ failure assessment MR-proADM: midregional proadrenomedullin; PCT: procalcitonin; Histones are represented by histone H2A, H2B, H3 and H4. H4 was also measured by an immunoassay (IA))

**Table 6**

| **Model** | **N** | **Events** | **L.R. χ²** | **df** | **p-value** | **C index** | **HR [95 % Cl] per ...** | |
|---|---|---|---|---|---|---|---|---|
| | | | | | | | **... IQR** | **... 2fold change** |
| **H2B** | 64 | 16 | 22.32 | 3 | <0.001 | 0.815 | 4.79 [2.44-9.43] | 1.60 [1.31-1.96] |
| **H2A** | 64 | 16 | 22.28 | 3 | <0.001 | 0.811 | 4.35 [2.31-8.20] | 1.61 [1.31-1.98] |
| **H4** | 64 | 16 | 21.99 | 3 | <0.001 | 0.812 | 4.53 [2.33-8.81] | 1.60 [1.30-1.97] |
| **H4 IA** | 61 | 16 | 21.95 | 3 | <0.001 | 0.803 | 5.52 [2.24-13.58] | 1.64 [1.26-2.13] |
| **H3** | 64 | 16 | 17.59 | 3 | 0.001 | 0.767 | 7.95 [2.70-23.45] | 1.40 [1.17-1.67] |
| **SAPS II** | 64 | 16 | 15.16 | 3 | 0.002 | 0.755 | 4.76 [1.99-11.38] | 1.02 [1.01-1.03] |
| **MR-proADM** | 64 | 16 | 10.20 | 3 | 0.017 | 0.737 | 3.15 [1.38-7.17] | 1.88 [1.20-2.96] |
| **SOFA** | 64 | 16 | 9.84 | 3 | 0.020 | 0.722 | 3.36 [1.42-7.95] | 1.06 [1.02-1.11] |
| **Aldolase B** | 64 | 16 | 7.30 | 3 | 0.063 | 0.644 | 2.05 [1.15-3.64] | 1.25 [1.05-1.50] |
| **PCT** | 64 | 16 | 4.75 | 3 | 0.191 | 0.661 | 1.95 [0.92-4.14] | 1.15 [0.98-1.34] |

References cited herein:
Albrich, W. C. and S. Harbarth (2015). " Intensive Care Med 41(10): 1739-1751.
Bone, R. C., R. A. Balk, et al. (1992). Chest 101(6): 1644-1655.
Bouch, D. C. and J. P. Thompson (2008). Continuing Education in Anaesthesia, Critical Care & Pain 8(5): 181-185.
Breslow, M. J. and O. Badawi (2012). Chest 141(1): 245-252.
Ferreira, A. M. and Y. Sakr (2011). Semin Respir Crit Care Med 32(5): 543-551.
Halpern, N. A. and S. M. Pastores (2010). Crit Care Med 38(1): 65-71.
Kaneko-Wada Fde, J., G. Dominguez-Cherit, et al. (2015). Gac Med Mex 151(5): 628-634.
Le Gall, J. R., S. Lemeshow, et al. (1993). JAMA 270(24): 2957-2963.
Mayr, V. D., M. W. Dunser, et al. (2006). Crit Care 10(6): R154.
Vincent, J. L. (2008). Langenbecks Arch Surg 393(6): 817-824.
Vincent, J. L., R. Moreno, et al. (1996). Intensive Care Med 22(7): 707-710.
Kutcher et al. (2012). Journal of Trauma and Acute Care Surgery 73(6): 1389-1394.
Ekaney et al. (2014). Critical Care 18(5): 1-9.
Abrams et al. (2013). American Journal of Respiratory and Critical Care Medicine 187(2): 160-169.
Potocki et al. (2012). Current Heart Failure Reports 9(3): 244-251.
Artunc et al. (2014). PLOS ONE 9(1): 1-8.
Meinders et al. (2012). European Heart Journal 33, Suppl. 1: 130.
Wildhagen et al. (2015). Thrombosis Research 136(3): 542-547.
De La Torre-Prados et al. (2016). Minerva Anestesiologica 82(7): 760-766.

## Claims

1. A method for the prognosis of mortality of a subject, wherein said subject is a critically ill patient, and wherein said method comprises
(i) determining a level of at least one histone in a blood, blood plasma, or blood serum sample of said subject; and
(ii) determining a level of proadrenomedullin (proADM) in a blood, blood plasma or blood serum sample of said subject; wherein
(i1) said level of at least one histone is compared to a reference level of at least one histone; and
(iii) said level of proADM is compared to a reference level of proADM; and
(iii) wherein said mortality of said subject is identified based on the comparison in step (i1) and (ii1), and wherein said level of at least one histone and said level of proADM are indicative of said mortality occurring within 7 days.

2. The method of claim 1, wherein
(i) an increase in the level of at least one histone as compared to the reference level of at least one histone is indicative of mortality of said subject; and/or
(ii) an increase in the level of proADM as compared to the reference level of proADM is indicative of mortality of said subject.

3. The method of any one of claims 1 or 2, wherein said reference level of at least one histone and said reference level of proADM are a level of at least one histone and a level of proADM of at least one reference subject, and wherein the reference subject(s) is/are healthy subject(s) and/or subject(s) in which no mortality occurs within 7 days.

4. The method of any one of claims 1 to 3, wherein proADM is midregional proadrenomedullin (MR-proADM) and wherein said at least one histone is histone H2B, histone H4, histone H2A and/or histone H3.

5. The method of any one of claims 1 to 4, wherein said method further comprises determining at least one marker and/or parameter of said subject selected from the group consisting of a level of aldolase B in said sample, a level of copeptin in said sample, a level of lactate in said sample, a level of procalcitonin (PCT) in said sample, the sequential organ failure assessment score (SOFA score) of said subject, the simplified acute physiology score II (SAPSII score), the Acute Physiology and Chronic Health Evaluation II (APACHE II) score of said subject and a level of the soluble fms-like tyrosine kinase-1 (sFlt-1) in said sample.

6. The method of any one of claims 1 to 5, wherein said subject suffers from a disease or medical condition and wherein said disease or medical condition is selected from the group consisting of respiratory disease, urinary tract infection, an inflammatory response related to infective and non-infective etiologies, systemic inflammatory response syndrome (SIRS), sepsis, severe sepsis, septic shock, organ failure(s), cardiovascular disease, diabetes mellitus, malignancy, liver disease, renal disease and immunodepression.

7. The method of any one of claims 1 to 6, wherein said subject is admitted to an intensive care unit.

8. The method of any one of claims 1 to 7, wherein said subject suffers from a respiratory disease, urinary tract infection or malignancy.

9. The method of any one of claims 1 to 8, wherein said level of at least one histone and/or of proADM is/are determined using a method selected from the group consisting of mass spectrometry (MS), luminescence immunoassay (LIA), radioimmunoassay (RIA), chemiluminescence- and fluorescence- immunoassays, enzyme immunoassay (EIA), Enzyme-linked immunoassays (ELISA), luminescence-based bead arrays, magnetic beads based arrays, protein microarray assays, rapid test formats, and rare cryptate assay.

10. The method of any one of claims 1 to 9,
(i) wherein said at least one histone is histone H2B and wherein at least a peptide of the sequence spanning amino acid residues 41 to 69 of histone H2B according to SEQ ID NO: 4 is determined;
(ii) wherein said at least one histone is histone H4 and wherein at least a peptide of the sequence spanning amino acid residues 22 to 102 of histone H4 according to SEQ ID NO:1 is determined;
(iii) wherein said at least one histone is histone H2A and wherein at least a peptide of the sequence spanning amino acid residues 20 to 118 of histone H2A according to SEQ ID NO: 2 is determined; and/or
(iv) wherein said at least one histone is histone H3 and wherein at least a peptide of the sequence spanning amino acid residues 27 to 62 of histone H3 according to SEQ ID NO: 3 is determined.

11. The use of a kit in the method of any one of the claims 1 to 10, wherein said kit comprises
(i) detection reagents for determining the level of at least one histone in said sample of said subject, and reference data including said reference level of at least one histone, and wherein an increase in the level of at least one histone in said sample of said subject as compared to said reference level of at least one histone is indicative of mortality of said subject occurring within 7 days; and
(ii) detection reagents for determining the level of proADM in said sample of said subject, and reference data including said reference level of proADM, and wherein an increase in the level of proADM in said sample of said subject as compared to said reference level of proADM is indicative of mortality of said subject occurring within 7 days.

## Patentansprüche

1. Ein Verfahren zur Prognose der Mortalität eines Subjekts, wobei das Subjekt ein kritisch kranker Patient ist und wobei das genannte Verfahren folgendes umfasst
(i) Bestimmen des Spiegels von mindestens einem Histon in einer Blut-, Blutplasma- oder Blutserumprobe des genannten Subjekts; und
(ii) Bestimmen des Proadrenomedullin-Spiegels (proADM) in einer Blut-, Blutplasma- oder Blutserumprobe des genannten Subjekts; wobei
(i1) der Spiegels von mindestens einem Histon mit einem Referenzspiegel von mindestens einem Histon verglichen wird; und
(ii1) der Spiegel von proADM mit einem proADM-Referenzspiegel verglichen wird; und
(iii) wobei die Mortalität des Subjekts auf der Grundlage des Vergleichs in Schritt (i1) und (ii1) ermittelt wird, und wobei der Spiegel von mindestens einem Histon und der proADM-Spiegel ein Hinweis auf eine innerhalb von 7 Tagen eintretende Mortalität ist.

2. Verfahren nach Anspruch 1, wobei:
(i) ein Anstieg des Spiegels von mindestens einem Histon im Vergleich zum Referenzspiegel von mindestens einem Histon auf die Mortalität des Subjekts hinweist; und/oder
ein Anstieg des proADM-Spiegels im Vergleich zum proADM-Referenzspiegel auf die Mortalität des Subjekts hinweist.

3. Das Verfahren nach einem der Ansprüche 1 oder 2, wobei der genannte Referenzspiegel von mindestens einem Histon und der genannte proADM-Referenzspiegel ein Spiegel von mindestens einem Histon und einem proADM-Spiegel von mindestens einem Referenzsubjekt(e) sind, und wobei der/die Referenzsubjekt(e) ein gesunder/gesunde Subjekt(e) ist/sind, und/oder ein bzw. mehrere Subjekt(e), bei dem/denen innerhalb von 7 Tagen keine Mortalität aufgetreten ist/sind.

4. Das Verfahren nach einem der Ansprüche 1 bis 3, wobei das ProADM mittelregionales Proadrenomedullin (MR-proADM) ist und wobei mindestens eines dieser genannten Histone ein Histon H2B, Histon H4, Histon H2A und/oder Histon H3 ist.

5. Das Verfahren nach einem der Ansprüche 1 bis 4, wobei das genannte Verfahren ferner die Bestimmung von mindestens einem Marker und/oder Parameter des Subjekts umfasst, ausgewählt aus der Gruppe, bestehend aus:
einem Aldolase-B-Spiegel in der Probe, einen Copeptin-Spiegel in der Probe, einen Laktatspiegel in der Probe, einen Procalcitoninspiegel (PCT) in der Probe, den Sequential Organ Failure Assessment Score (SOFA score) des genannten Subjekts, den Simplified Acute Physiology Score II (SAPSII-Score), den Acute Physiology and Chronic Health Evaluation II (APACHE II) Score des genannten Subjekts und den Spiegel der löslichen fms-ähnlichen Tyrosinkinase-1 (sFlt-1) in der Probe.

6. Das Verfahren nach einem der Ansprüche 1 bis 5, wobei das Subjekt an einer Krankheit oder einem medizinischen Zustand leidet und wobei die Krankheit oder der medizinische Zustand aus der Gruppe ausgewählt wird, die aus Atemwegserkrankungen, Hamwegsinfektionen, einer Entzündungsreaktion im Zusammenhang mit infektiösen und nicht-infektiösen Ursachen, eines systemischen Entzündungsreaktionssyndroms (SIRS), einer Sepsis, schweren Sepsis, einem septischem Schock, Organversagen, einer Herz-Kreislauf-Erkrankung, Diabetes mellitus, einer bösartigen Tumorerkrankung (Malignität), Lebererkrankung, Nierenerkrankung und Immunsuppression bestehen.

7. Das Verfahren nach einem der Ansprüche 1 bis 6, wobei das genannte Subjekt in einer Intensivstation aufgenommen ist.

8. Das Verfahren nach einem der Ansprüche 1 bis 7, wobei das genannte Subjekt an einer Atemwegserkrankung, Harnwegsinfektion oder einer bösartigen Tumorerkrankung (Malignität) leidet.

9. Verfahren nach einem der Ansprüche 1 bis 8, wobei der genannte Spiegel von mindestens einem Histon und oder der proADM-Spiegel unter Verwendung eines Verfahrens bestimmt wird, das aus folgender Gruppe ausgewählt ist: Massenspektrometrie (MS), Lumineszenzimmunoassay (LIA), Radioimmunoassay (RIA), Chemilumineszenz- und Fluoreszenzimmunoassays, Enzymimmunoassay (EIA), Enzym-gekoppelte Immunoassays (ELISA), Lumineszenz-basierte Bead-Arrays, Magnetkügelchen-basierte Arrays, Protein-Microarray-Assays, Schnelltestformate und seltenen Kryptat-Assays.

10. Verfahren nach einem der Ansprüche 1 bis 9,
(i) wobei mindestens ein Histon das Histon H2B ist und wobei mindestens ein Peptid der Sequenz, die die Aminosäurereste 41 bis 69 von Histon H2B gemäß SEQ ID NO: 4 umfasst, bestimmt wird;
(ii) wobei mindestens ein Histon das Histon H4 ist und wobei mindestens ein Peptid der Sequenz, die die Aminosäurereste 22 bis 102 von Histon H4 gemäß SEQ ID NO: 1 umfasst, bestimmt wird;
(iii) wobei mindestens ein Histon das Histon H2A ist und wobei mindestens ein Peptid der Sequenz, die die Aminosäurereste 20 bis 118 von Histon H2A gemäß SEQ ID NO: 2 umfasst, bestimmt wird; und/oder
(iv) wobei mindestens ein Histon das Histon H3 ist und wobei mindestens ein Peptid der Sequenz, die die Aminosäurereste 27 bis 62 von Histon H3 gemäß SEQ ID NO: 3 umfasst, bestimmt wird.

11. Verwendung eines Kits in dem Verfahren nach einem der Ansprüche 1 bis 10, wobei der genannte Kit Folgendes umfasst:
(i) Nachweisreagenzien zur Bestimmung des Spiegels von mindestens einem Histon in der genannten Probe des Subjekts, und Referenzdaten, einschließlich des Referenzspiegels von mindestens einem Histon, und wobei ein Anstieg des Spiegels von mindestens einem Histon in der Probe des Subjekts im Vergleich zum Referenzspiegel von mindestens einem Histon darauf hinweist, dass die Mortalität des genannten Subjekts innerhalb von 7 Tagen eintritt; und
(ii) Nachweisreagenzien zur Bestimmung des proADM-Spiegels in der Probe des Subjekts, und Referenzdaten, einschließlich des proADM-Referenzspiegels, und wobei ein Anstieg des proADM-Spiegels in der Probe des Subjekts im Vergleich zum proADM-Referenzspiegel ein Hinweis auf eine innerhalb von 7 Tagen eintretende Mortalität ist.

## Revendications

1. Procédé destiné au pronostic de la mortalité d'un sujet, où le patient est un patient gravement malade, et où la méthode comprend
(i) la détermination du niveau d'au moins une histone dans un échantillon de sang, de plasma sanguin ou de sérum sanguin du sujet ; et
(ii) la détermination d'un niveau de pro-adrénomédulline (proADM) dans un échantillon de sang, de plasma sanguin ou de sérum sanguin du sujet ; où
(i1) le niveau d'au moins une histone est comparé à un niveau de référence d'au moins une histone ; et
(ii1) le niveau de proADM est comparé à un niveau de référence de proADM ; et
(iii) où la mortalité du sujet est identifiée sur la base de la comparaison des étapes (i1) et (ii1), et où le niveau d'au moins une histone et le niveau de proADM sont indicatifs de la mortalité à 7 jours.

2. Procédé selon la revendication 1, où
(i) une augmentation du niveau d'au moins une histone par rapport au niveau de référence d'au moins une histone indique la mortalité dudit sujet ; et/ou
(ii) une augmentation du niveau de proADM par comparaison avec le niveau de référence de proADM est indicative de la mortalité du sujet.

3. Le procédé selon l'une des revendications 1 ou 2, où le niveau de référence d'au moins une histone et le niveau de référence de proADM sont un niveau d'au moins une histone et un niveau de proADM d'au moins un sujet de référence, et où le ou les sujets de référence sont un ou des sujets en bonne santé et/ou un ou des patients chez lesquels aucune mortalité ne survient à 7 jours.

4. Le procédé selon l'une des revendications 1 à 3, où la proADM est la pro-adrénomédulline mi-régionale (MR-proADM) et où au moins une histone est l'histone H2B, l'histone H4, l'histone H2A et/ou l'histone H3.

5. Le procédé selon l'une des revendications 1 à 4, où le procédé comprend en outre la détermination d'au moins un marqueur et/ou un paramètre du sujet choisi dans le groupe composé d'un niveau d'aldolase B dans l'échantillon, un niveau de copeptine dans l'échantillon, un taux de lactate dans l'échantillon, un niveau de procalcitonine (PCT) dans l'échantillon, le score séquentiel d'évaluation
de défaillance d'organe (score SOFA) du sujet, le score de physiologie aigu simplifié (score SAPSII), le score de physiologie aiguë simplifié Il (score PASII), le score d'évaluation de la physiologie aiguë et de la santé chronique II (APACHE II) et un niveau de tyrosine kinase soluble de type fms-1 (sFlt-1) dans l'échantillon.

6. Procédé selon l'une des revendications 1 à 5, où le sujet est atteint d'une maladie ou d'une affection médicale et où la maladie ou l'affection médicale est choisie dans le groupe composé de maladies respiratoires, infections urinaires, réponse inflammatoire liée à des étiologies infectieuses et non infectieuses, syndrome de réponse inflammatoire systémique (SRIS), sepsis, sepsis sévère, choc septique, défaillance(s) d'organe(s), maladie cardiovasculaire, diabète de type 2, tumeur maligne, maladie du foie, maladie rénale et immunodépression.

7. Procédé selon l'une des revendications 1 à 6, où le sujet est admis dans une unité de soins intensifs.

8. Procédé selon l'une des revendications 1 à 7, où le sujet est atteint d'une maladie respiratoire, d'une infection urinaire ou d'une tumeur maligne.

9. Procédé selon l'une des revendications 1 à 8, où le niveau d'au moins une histone et/ou de proADM est déterminé au moyen d'un procédé choisi dans le groupe composé de spectrométrie de masse (MS), immunodosage par luminescence (LIA), dosage radio-immunologique (RIA), immunodosage par chimiluminescence et fluorescence, immunodosage enzymatique (EIA), immunodosages liés aux enzymes (ELISA), réseaux de billes basés sur la luminescence, réseaux basés sur billes magnétiques, dosages de microréseaux de protéines, formats de tests rapides et dosage de cryptates rares.

10. Le procédé selon l'une des revendications 1 à 9,
(i) où au moins une histone est l'histone H2B et où au moins une peptide de la séquence couvrant les résidus d'acides aminés 41 à 69 de l'histone H2B selon SEQ ID No:4 est déterminée ;
(ii) où au moins une histone est l'histone H4 et où au moins un peptide de la séquence couvrant les résidus d'acide aminé 22 à 102 de l'histone H4 selon SEQ ID NO:1 est déterminé ;
(iii) où au moins une histone est l'histone H2A et où au moins un peptide de la séquence couvrant les résidus d'acide aminé 20 à 118 de l'histone H2A selon SEQ ID NO:2 est déterminé ; et/ou
(iv) où au moins une histone est l'histone H3 et où au moins un peptide de la séquence couvrant les résidus d'acides aminés 27 à 62 de l'histone H3 selon SEQ ID NO: 3 est déterminé.

11. L'utilisation d'un kit dans le procédé de l'une des réclamations 1 à 10, où le kit comprend
(i) des réactifs de détection pour déterminer le niveau d'au moins une histone dans l'échantillon du sujet, et des données de référence comprenant le niveau de référence d'au moins une histone, et dans laquelle une augmentation du niveau d'au moins une histone dans l'échantillon du sujet par comparaison au niveau de référence d'au moins une histone est indicative de la mortalité du sujet survenant dans les 7 jours; et
(ii) des réactifs de détection pour déterminer le niveau de proADM dans l'échantillon du sujet, et des données de référence comprenant le niveau de référence de proADM, et dans lesquelles une augmentation du niveau de proADM dans l'échantillon du sujet par comparaison au niveau de référence de proADM est indicative de la mortalité du sujet survenant dans les 7 jours.
